# EUROPÄISCHE PATENTSCHRIFT

(11) **EP 3 115 082 B1**
(45) Veröffentlichungstag und Bekanntmachung des Hinweises auf die Patenterteilung: **18.04.2018**
(21) Anmeldenummer: 16177495.5
(22) Anmeldetag: 01.07.2016
(51) Int. Cl.: A61N 5/10, G21K 1/093

(54) **TEILCHENSTRAHL-THERAPIEANLAGE MIT SOLENOID-MAGNETEN**
PARTICLE BEAM THERAPY INSTALLATION WITH SOLENOID MAGNETS
INSTALLATION DE THÉRAPIE PAR IRRADIATION DE PARTICULES AVEC AIMANTS SOLÉNOÏDES

(30) Priorität: 08.07.2015 DE 102015111060
(43) Veröffentlichungstag der Anmeldung: 11.01.2017
(73) Patentinhaber: CRYOELECTRA GMBH, 42287 Wuppertal (DE)
(72) Erfinder: DREES, Jürgen, 42287 Wuppertal (DE); PIEL, Helmut, 42287 Wuppertal (DE)
(74) Vertreter: Cohausz & Florack

(56) Entgegenhaltungen:
- EP-A1- 3 086 325
- US-A1- 2009 090 871
- US-A1- 2012 313 003
- ROBIN D S ET AL: "Superconducting toroidal combined-function magnet for a compact ion beam cancer therapy gantry", NUCLEAR INSTRUMENTS & METHODS IN PHYSICS RESEARCH. SECTION A: ACCELERATORS, SPECTROMETERS, DETECTORS, AND ASSOCIATED EQUIPMENT, ELSEVIER BV * NORTH-HOLLAND, NL, Bd. 659, Nr. 1, 25. August 2011 (2011-08-25), Seiten 484-493, XP028101389, ISSN: 0168-9002, DOI: 10.1016/J.NIMA.2011.08.049 [gefunden am 2011-09-14]

## Beschreibung

Die Erfindung betrifft gemäß einem ersten Aspekt eine Teilchenstrahl-Therapieanlage mit einer Strahlerzeugungseinheit zur Erzeugung eines Strahls geladener Teilchen, insbesondere Ionen, vorzugsweise Protonen, und mit einem Strahlführungssystem. Die Erfindung betrifft zudem gemäß einem zweiten Aspekt ein vorteilhaftes Verfahren.

Eingangs genannte Teilchenstrahl-Therapieanlagen werden im Stand der Technik zur Strahlentherapie mit geladenen Teilchen, beispielsweise Ionen in Form von Protonen, verwendet. Dies bietet gegenüber der bisher üblichen Strahlentherapie mit Photonen wesentliche Vorteile für Patienten mit bestimmten Krebserkrankungen. Insbesondere ist eine Bestrahlung mit Ionen, insbesondere Protonen, vorteilhaft, da diese erst am Ende ihres Laufwegs im zu bestrahlenden Gewebe, im sogenannten Bragg-Peak, ihre maximale Ionisationsstärke, und damit einhergehend ihre größte Zerstörungskraft, beispielsweise für Tumorzellen, aufweisen. Auf diese Weise kann die Beeinträchtigung von gesundem Gewebe, welches dem zu behandelnden Gewebe im Laufweg des Strahls vorgelagert ist und von dem Strahl durchlaufen wird, reduziert werden. Zudem kann die Beeinträchtigung von gesundem Gewebe, welches dem Strahl nachgelagert ist, fast vollständig vermieden werden.

Die erfolgreiche Behandlung von Tumoren mit einem Teilchenstrahl, beispielsweise mittels des Raster-Scan-Therapieverfahrens, erfordert allerdings einen Teilchenstrahl mit präzisen Strahleigenschaften, insbesondere der Strahlposition oder des Strahlimpulses, sodass am Behandlungsort (am sogenannten Isozentrum) eine präzise Behandlung des Gewebes erfolgen kann und Behandlungsungenauigkeiten vermieden werden, sodass möglichste keine unerwünschte Bestrahlung von gesundem Gewebe (etwa angrenzende Organe) erfolgt.

Die Teilchenstrahl-Therapiesysteme aus dem Stand der Technik, das heißt die Gesamtsysteme und insbesondere die Strahlführungssysteme, nehmen allerdings vergleichsweise viel Platz ein. Zudem sind die Strahleigenschaften und die erreichbare Transmission des Teilchenstrahls bis zum Behandlungsort verbesserungsbedürftig. Aus der WO 2009/106603 A1 ist beispielsweise ein Teilchenstrahl-Therapiesystem bekannt, wobei ein Teilchenstrahl erzeugt und über ein Strahlführungssystem einem von mehreren Behandlungsräumen zugeführt wird. Es wird vorgeschlagen, zur Reduzierung des betrieblichen Aufwandes unterschiedliche Strahlführungssysteme vorzusehen, welche an bestimmte Strahleigenschaften angepasst sind. Dies kann durch die Verwendung einer einzigen Strahlerzeugungseinheit für mehrere Behandlungsorte zwar eine Platzersparnis bringen. Allerdings ist dies nur möglich, sofern mehrere Behandlungsorte vorgesehen werden und ohnehin viel Platz vorhanden ist. Zudem benötigen das Strahlführungssystem und die darin vorgesehenen Weichen weiterhin vergleichsweise viel Raum.
Aus der EP 2 268 359 B1 ist ebenfalls ein Teilchenstrahl-Therapiesystem bekannt, wobei zur Gewährleistung einer präzisen Strahlführung vorgeschlagen wird, mittels eines Strahlpositionsmonitors die Strahleigenschaften während der Behandlung im Halo-Bereich des Teilchenstrahls vorzunehmen. Dies ermöglicht zwar eine verbesserte Überprüfung der Strahleigenschaften. Allerdings sind die Strahleigenschaften weiterhin vorrichtungstechnisch begrenzt, sodass weiterhin das Bedürfnis besteht, die Strahleigenschaften zu verbessern. Zudem benötigt auch dieses System vergleichsweise viel Raum.

ROBIN D S ET AL: "Superconducting toroidal combined-function magnet for a compact ion beam cancer therapy gantry" definiert ein Teilchenstrahl-Therapiesystem wobei ein supraleitender Solenoid-Magnet als finaler 90°-Grad Umlenkmagnet eingesetzt wird. Hiervon ausgehend ist es Aufgabe der vorliegenden Erfindung, ein gattungsgemäßes Strahlführungssystem anzugeben, welches bei reduziertem Platzbedarf vergleichbare oder sogar verbesserte Strahleigenschaften bereitstellen kann. Weiterhin liegt der vorliegenden Erfindung die Aufgabe zugrunde, eine vorteilhafte Teilchenstrahl-Therapieanlage und ein vorteilhaftes Verfahren vorzuschlagen.

Bei einer gattungsgemäßen Teilchenstrahl-Therapieanlage wird die Aufgabe gemäß einem ersten Aspekt dadurch gelöst, dass das Stahlführungssystem in Richtung des Strahls geladener Teilchen gesehen hinter der Strahlerzeugungseinheit mindestens einen Solenoid-Magneten als Strahlformungseinheit aufweist, und der mindestens eine Solenoid-Magnet des Stahlführungssystems ein supraleitender Solenoid-Magnet ist.

Es hat sich gezeigt, dass durch das Vorsehen von einem oder mehr Solenoid-Magneten als Strahlformungseinheiten in dem Strahlführungssystem, wobei der eine Solenoid-Magnet oder die mehreren Solenoid-Magneten supraleitend sind, zum einen verbesserte Strahleigenschaften, etwa eine erhöhte Transmission bis zum Behandlungsort, und zum anderen eine Reduzierung des Platzbedarfs der Teilchenstrahl-Therapieanlage erreicht werden kann. Durch den mindestens einen Solenoid-Magneten kann insbesondere der Phasenraum des Strahls geladener Teilchen optimal für die Weiterführung präpariert werden. Der mindestens eine Solenoid-Magnet wirkt wie eine Sammellinse und reduziert die Auffächerung des Strahls geladener Teilchen. Es hat sich zudem gezeigt, dass der Strahl geladener Teilchen auch bezüglich der Rotationssymmetrie gute Strahleigenschaften aufweist. Die Kombination aus vorteilhaften Strahleigenschaften und reduzierten Raumbedarf war im Stand der Technik in dieser Kombination bisher nicht erreicht. Es hat sich beispielsweise gezeigt, dass es möglich ist, die Länge der Teilchenstrahl-Therapieanlage bis zum Behandlungsort gemessen auf weniger als 16, insbesondere weniger 14 m zu reduzieren.

Das Strahlführungssystem dient insbesondere dazu, den Strahl geladener Teilchen mit bestimmten Eigenschaften bis zum Behandlungsort zu transportieren. Das heißt, das Strahlführungssystem kann insbesondere der Formung des Strahls geladener Teilchen und der Ablenkung des Stahls geladener Teilchen dienen. Ebenfalls kann das Strahlführungssystem dazu dienen die Eigenschaften des Strahls geladener Teilchen zu überprüfen, beispielsweise indem Strahlmonitore in dem Strahlführungssystem vorgesehen werden. Ebenfalls kann das Strahlführungssystem dazu dienen oder es ermöglichen, den Behandlungsort von der Strahlerzeugungseinheit abzuschirmen, beispielsweise indem eine Driftstrecke vorgesehen wird, welche das Vorsehen einer Abschirmung, etwa in Form einer Betonwand, ermöglicht.

Die Strahlerzeugungseinheit ist beispielsweise eine Beschleunigereinrichtung, beispielsweise eine Beschleunigereinrichtung, welche einen Strahl geladener Teilchen mit konstanter Energie erzeugt, beispielsweise ein Zyklotron. Es ist jedoch auch denkbar, dass die Strahlerzeugungseinrichtung eine Beschleunigereinrichtung ist, welche einen Strahl geladener Teilchen mit variabel einstellbarer Energie erzeugt, beispielsweise ein Synchrotron. Bevorzugt wird eine Strahlerzeugungseinheit verwendet, welche geladene Teilchen mit einer (mittleren) kinetischen Energie von mehr als 200 MeV emittiert, beispielsweise zwischen 200 MeV und 300 MeV, insbesondere zwischen 210 MeV und 250 MeV. Bevorzugt wird eine Strahlerzeugungseinheit mit einem Durchmesser von weniger als 4 m verwendet, um den Platzbedarf der Teilchenstrahl-Therapieanlage gering zu halten.

Der Strahl von Teilchen ist bevorzugt ein Ionenstrahl, insbesondere ein Protonenstrahl. Das Strahlführungssystem ist beispielsweise dazu ausgelegt, den Strahl geladener Teilchen mit Energien von 60 bis 210 MeV gezielt zu führen. 1 MeV entspricht ungefähr 1,6 x 10⁻¹³ Joule. Dabei wird unter der Energie der Teilchen insbesondere die mittlere oder maximale kinetische Energie der Teilchen verstanden.

Unter einem Solenoid-Magneten wird insbesondere eine Zylinderspule zum Erzeugen eines (räumlich möglichst konstanten) Magnetfeldes verstanden. Der Strahl geladener Teilchen wird dabei durch das innere des Solenoid-Magneten geführt. Das Magnetfeld verläuft dabei im Inneren des Solenoid-Magneten insbesondere im Wesentlichen parallel zum Strahl geladener Teilchen. Beispielsweise liegen die Leiterwicklungen (etwa Drahtwicklungen) des Solenoid-Magneten auf einem Zylindermantel und sind dünn gegenüber dem Zylinderdurchmesser.

Unter supraleitenden Solenoid-Magneten wird insbesondere verstanden, dass das Magnetfeld zumindest teilweise durch einen Stromfluss durch ein supraleitendes Material, das heißt einen Supraleiter, erzeugt wird. Unter einem Supraleiter wird ein Material verstanden, dessen elektrischer Widerstand beim Unterschreiten einer Sprungtemperatur (abrupt) auf null fällt. Hierdurch können höhe Ströme durch den Solenoid geführt werden, was hohe Magnetfelder und damit eine effektive Strahlformung ermöglicht.

Als Supraleiter können hierbei etwa metallische Supraleiter, wie etwa NbTi oder Nb₃SN verwendet werden.

Dass der mindesten eine Solenoid-Magnet als Strahlformungseinheit vorgesehen ist, bedeutet, dass eine Eigenschaft des Strahls geladener Teilchen durch den mindestens einen Solenoid-Magneten beeinflusst werden kann. Beispielsweise kann die Divergenz des Strahls geladener Teilchen verringert werden. Insbesondere wird durch den mindestens einen Solenoid-Magneten der Strahl geladener Teilchen nicht wie etwa durch eine Strahlablenkeinheit abgelenkt, sondern der Strahl geladener Teilchen bewegt sich im Mittel in die gleiche Richtung weiter.

Dass mindesten ein Solenoid-Magnet vorgesehen ist, bedeutet, dass bevorzugt auch mehr als ein supraleitender Solenoid-Magnet vorgesehen sein kann. Dabei können die Solenoid-Magnete voneinander abweichend oder identisch ausgebildet sein.

Gemäß einer bevorzugten Ausgestaltung der erfindungsgemäßen Teilchenstrahl-Therapieanlage ist mindestens ein Solenoid-Magnet des Strahlführungssystems unmittelbar hinter der Strahlerzeugungseinheit vorgesehen. Dadurch, dass der mindestens eine Solenoid-Magnet unmittelbar hinter der Strahlerzeugungseinheit vorgesehen ist, können die Strahleigenschaften vergleichsweise früh beeinflusst werden und eine Fokussierung des Strahls geladener Teilchen auf nachfolgend im Strahl angeordnete Elemente erreicht werden.

Dass mindestens ein Solenoid-Magnet unmittelbar hinter der Strahlerzeugungseinheit vorgesehen ist, bedeutet insbesondere, dass keine anderen Strahlformungseinheiten und/oder Strahlablenkeinheiten zwischen der Strahlerzeugungseinheit und dem einen Solenoid-Magneten vorgesehen sind. Es ist jedoch denkbar, dass weiterhin etwa ein Strahlmonitor zur Kontrolle der Strahleigenschaften zwischen der Strahlerzeugungseinheit und dem einen Solenoid-Magneten vorgesehen sein kann.

Gemäß einer bevorzugten Ausgestaltung der erfindungsgemäßen Teilchenstrahl-Therapieanlage weist das Strahlführungssystem eine Energiekorrektureinheit zur Einstellung der Energie der geladenen Teilchen des Strahls geladener Teilchen auf. Dabei ist mindestens ein Solenoid-Magnet des Strahlführungssystems in Richtung des Strahls geladener Teilchen gesehen zwischen der Strahlerzeugungseinheit und der Energiekorrektureinheit vorgesehen und/oder mindestens ein Solenoid-Magnet des Strahlführungssystems in Richtung des Strahls geladener Teilchen gesehen hinter der Energiekorrektureinheit vorgesehen.

Durch die Energiekorrektureinheit wird eine flexible Anpassung der Energie der geladenen Teilchen des Strahls geladener Teilchen ermöglicht. Beispielsweise ist es möglich, eine Strahlerzeugungseinheit (zum Beispiel ein Zyklotron) zu verwenden, welche geladene Teilchen mit im Wesentlichen konstanter (mittlerer) Energie emittiert, ohne darauf verzichten zu müssen, die Energie der geladenen Teilchen des Strahls geladener Teilchen flexibel einstellen zu können. Beispielsweise ist die Energiekorrektureinheit zur Reduzierung der Energie der geladenen Teilchen des Strahls geladener Teilchen eingerichtet. Beispielsweise ist die Energiekorrektureinheit ein Degrader. Dadurch braucht lediglich eine Strahlerzeugungseinheit, welche Teilchen mit ausreichender maximaler Energie emittiert, vorgesehen sein. Mittels der Energiekorrektureinheit kann die Energie der geladenen Teilchen des Strahls geladener Teilchen dann nach Bedarf eingestellt werden.

Beispielsweise kann die Energie der geladenen Teilchen des Strahls geladener Teilchen reduziert werden, indem die (mittlere) kinetische Energie der geladenen Teilchen reduziert wird. Durch die Energiekorrektureinheit kann beispielsweise ein Abbremsen der geladenen Teilchen auf eine wählbare mittlere kinetische Energie erfolgen.

Es hat sich gezeigt, dass das Strahlführungssystem so kompakt gebaut werden kann, dass beispielsweise der Behandlungsort weniger als 10 m, insbesondere etwa 9 m vom Ende der Energiekorrektureinheit entfernt positioniert werden kann.

Beispielsweise kann eine Strahlerzeugungseinheit verwendet werden, welche geladene Teilchen mit einer kinetischen Energie von mehr als 200 MeV emittiert, beispielsweise zwischen 200 und 300 MeV, insbesondere zwischen 210 und 250 MeV. Die Energiekorrektureinheit kann beispielsweise dergestalt sein, dass die kinetische Energie der geladenen Teilchen beispielsweise auf unter 200 MeV und/oder auf unter 100 MeV reduziert werden kann.

Ist mindestens ein Solenoid-Magnet des Strahlführungssystems in Richtung des Strahls geladener Teilchen gesehen zwischen der Strahlerzeugungseinheit und der Energiekorrektureinheit vorgesehen, kann die Transmission des Strahls geladener Teilchen bis zum Behandlungszentrum erhöht werden. Dies ist darauf zurückzuführen, dass eine Abbildung des von der Strahlerzeugungseinheit emittierten Phasenraums auf die (unmittelbar) dahinter angeordnete Energiekorrektureinheit derart erfolgen kann, dass ein möglichst großer (im Optimalfall der gesamte) Teil des Phasenraums geladene Teilchen enthält, welche zur Transmission bis zum Behandlungsort beitragen. Mit anderen Worten kann der Strahl geladener Teilchen vorteilhaft auf die Energiekorrektureinheit fokussiert oder abgebildet werden. Dadurch kann ein hoher Anteil der geladenen Teilchen die Energiekorrektureinheit passieren und zu einer hohen Transmission durch die Energiekorrektureinheit beitragen. Bevorzugt ist genau ein Solenoid-Magnet zwischen der Strahlerzeugungseinheit und der Energiekorrektureinheit vorgesehen.

Trotz des Vorsehens eines Solenoid-Magneten des Strahlführungssystems zwischen der Strahlerzeugungseinheit und der Energiekorrektureinheit, kann die Strecke von der Strahlerzeugungseinheit bis zum Ende der Energiekorrektureinheit vorteilhaft weniger als 2 m betragen.

Ist mindestens ein Solenoid-Magnet des Strahlführungssystems in Richtung des Strahls geladener Teilchen gesehen hinter der Energiekorrektureinheit vorgesehen, kann einer Divergenz des Strahl geladener Teilchen nach der Energiekorrektureinheit entgegengewirkt werden und der Strahl geladener Teilchen kann vorteilhaft auf nachfolgende im Strahl angeordnete Elemente fokussiert oder abgebildet werden. Dadurch kann ein hoher Anteil der geladenen Teilchen das Strahlführungssystem bis zum Behandlungszentrum passieren und zu einer hohen Transmission durch das Strahlführungssystem beitragen. Bevorzugt sind keine anderen Strahlformungseinheiten, Strahlablenkeinheiten und/oder anderen magnetischen Einheiten zwischen der Energiekorrektureinheit und dem einen Solenoid-Magneten vorgesehen. Es ist jedoch denkbar, dass beispielsweise ein Kollimator zwischen der Energiekorrektureinheit und dem einen Solenoid-Magneten vorgesehen ist. Bevorzugt ist genau ein Solenoid-Magnet in Richtung des Strahls geladener Teilchen gesehen hinter der Energiekorrektureinheit vorgesehen.

Trotz des Vorsehens eines Solenoid-Magneten des Strahlführungssystems hinter der Energiekorrektureinheit kann die Strecke vom Ende der Energiekorrektureinheit bis zum Behandlungsort bevorzugt weniger als 10 m, insbesondere etwa 9 m betragen.

Bevorzugt weist die Energiekorrektureinheit mindestens ein quer zum Strahl geladener Teilchen verschiebbares blockförmiges Energiekorrekturelement und mindestens ein quer zum Strahl geladener Teilchen verschiebbares keilförmiges Energiekorrekturelement auf.

Ein blockförmiges Energiekorrekturelement umfasst beispielswiese eine Eintrittsseite und eine Austrittsseite für den Strahl geladener Teilchen, welche im Wesentlichen parallel verlaufen. Ein blockförmiges Energiekorrekturelement kann beispielsweise ein Quader sein.

Ein keilförmiges Energiekorrekturelement umfasst beispielsweise eine Eintrittsseite und eine Austrittsseite für den Strahl geladener Teilchen, welche schräg, das heißt nicht parallel, zueinander verlaufen.

Insbesondere durch die vorteilhafte Kombination mindestens eines blockförmigen und mindestens eines keilförmigen Energiekorrekturelements kann eine kompakte und präzise Reduzierung der Energie der geladenen Teilchen des Strahls geladener Teilchen erreicht werden. Insbesondere aufgrund der Kompaktheit (d.h. eine kurze von dem Strahl geladener Teilchen zu durchlaufende Strecke) kann durch eine solche Energiekorrektureinheit ein übermäßiges Aufweiten des Phasenraums des Strahls geladener Teilchen vermieden werden. Das blockförmige Energiekorrekturelement dient beispielsweise einer ersten groben Einstellung der Energie der geladenen Teilchen des Strahls geladener Teilchen. Beispielsweise kann die Energie der geladenen Teilchen des Strahls geladener Teilchen auf diskrete Werte eingestellt werden. Das keilförmige Energiekorrekturelement dient beispielsweise einer im Vergleich zur ersten Einstellung feineren Einstellung der Energie der geladenen Teilchen des Strahls geladener Teilchen. Beispielsweise kann die Energie der geladenen Teilchen des Strahls geladener Teilchen (beispielsweise in einem bestimmten Bereich) kontinuierlich eingestellt werden. Beispielsweise wird durch die Verschiebung des keilförmigen Energiekorrekturelements quer zum Strahl geladener Teilchen die in Richtung des Strahls geladener Teilchen gesehene Ausdehnung des keilförmigen Energiekorrekturelements im Bereich des Strahls geladener Teilchen verändert. Durch das Anordnen der blockförmigen Energiekorrekturelemente in Richtung des Strahls geladener Teilchen gesehen vor den keilförmigen Energiekorrekturelementen, können letztere entsprechend kürzer ausgebildet sein, da die Energie nur über einen kleineren Bereich eingestellt zu werden braucht.

Beispielsweise ist das mindestens eine blockförmige Energiekorrekturelement in eine Richtung (zum Beispiel in x-Richtung) quer zum Strahl geladener Teilchen verschiebbar. Beispielsweise ist das mindestens eine keilförmige Energiekorrekturelement in der gleichen und/oder in der dazu senkrechten transversalen Richtung (z.B. in x-Richtung und/oder in y-Richtung) quer zum Strahl geladener Teilchen verschiebbar.

Vorzugsweise sind das mindestens eine blockförmige Energiekorrekturelement und das mindestens eine keilförmige Energiekorrekturelement im Wesentlichen senkrecht zum Strahl geladener Teilchen verschiebbar.

Bevorzugt weist die Energiekorrektureinheit eine Mehrzahl von quer zum Strahl geladener Teilchen verschiebbaren blockförmigen Energiekorrekturelementen auf, welche unterschiedliche Einstellungen der Energie des Strahls geladener Teilchen ermöglichen.

Durch die Mehrzahl von (vorzugsweise unterschiedlichen) blockförmigen Energiekorrekturelementen kann eine flexible Einstellung der Energie der geladenen Teilchen des Strahls geladener Teilchen über einen weiten Energiebereich ermöglicht werden. Die blockförmigen Energiekorrekturelemente können beispielsweise in Richtung des Strahls geladener Teilchen gesehen unterschiedliche Ausdehnungen aufweisen und/oder aus unterschiedlichen Materialien bestehen.

Bevorzugt weist die Energiekorrektureinheit mehrere, insbesondere zwei quer zum Strahl geladener Teilchen verschiebbare keilförmige Energiekorrekturelemente auf.

Beispielsweise ist die Energiekorrektureinheit derart eingerichtet, dass die keilförmigen Energiekorrekturelemente gleichzeitig in den Strahl geladener Teilchen positioniert werden können. Beispielsweise ist ein keilförmiges Energiekorrekturelement aus einer ersten Richtung in den Strahl geladener Teilchen verschiebbar und ein weiteres keilförmiges Energiekorrekturelement aus einer zweiten Richtung (beispielsweise aus einer der ersten Richtung entgegengesetzten Richtung) in den Strahl geladener Teilchen verschiebbar.

Dadurch kann der Bereich, in dem eine Feinabstimmung möglich ist, erweitert werden. Zudem kann dadurch eine über den Querschnitt des Strahls geladener Teilchen asymmetrische Reduktion der Energie vermieden werden. Beispielsweise sind zwei keilförmige Energiekorrekturelemente hierzu spiegelsymmetrisch oder punktsymmetrisch zueinander angeordnet.

Bevorzugt ist die Energiekorrektureinheit zumindest teilweise aus Borcarbid hergestellt. Im Gegensatz zu im Stand der Technik verwendeten Materialien kann durch die Verwendung von Borcarbid (B₄C) eine kompaktere und sicherere Energiekorrektureinheit bereitgestellt werden. Der hohe Anteil an Bor (Ordnungszahl 5) und die hohe Dichte von Borcarbid reduzieren die Aufweitung des Phasenraums im Vergleich zu bisher verwendeten Materialien. Beispielsweise kann im Vergleich zu aus (ausschließlich) Graphit bestehenden Energiekorrektureinheiten eine um bis zu 30% kompaktere Energiekorrektureinheit bereitgestellt werden. Bevorzugt beträgt der Abstand vom Austrittsfenster der Strahlerzeugungseinheit (zum Beispiel ein Beschleuniger) bis zum Ende der Energiekorrektureinheit in Richtung des Strahls geladener Teilchen gesehen weniger als 2 m, insbesondere weniger als 1,5 m. Gleichzeitig kann durch die geringere Ausdehnung der Energiekorrektureinheit eine geringere Ausdehnung in Richtung des Strahls geladener Teilchen eine geringere Aufweitung des Strahls geladener Teilchen erreicht werden. Dies ermöglicht schließlich verbesserte Strahleigenschaften und verbesserte Transmissionseigenschaften der Teilchenstrahl-Therapieanlage. Zudem kann im Vergleich zu Beryllium enthaltenden Energiekorrektureinheit eine Energiekorrektureinheit mit geringerer Toxizität bereitgestellt werden.

Es ist ebenfalls möglich, eine Energiekorrektureinheit aus unterschiedlichen Materialien, beispielsweise Borcarbid und Graphit, vorzusehen. Beispielsweise können die blockförmigen und keilförmigen Energiekorrekturelemente aus unterschiedlichen Materialien bestehen.

Beispielsweise kann unter anderem durch den Einsatz einer derartigen Energiekorrektureinheit in Kombination mit supraleitenden Solenoid-Magneten der Strahl geladener Teilchen mit einer Energie von 215MeV auf 70 MeV abgebremst werden, wobei noch eine Transmission von über 3 % am Behandlungsort erreicht werden kann. Beispielsweise kann bei der Verwendung von mindestens einem supraleitenden Solenoid-Magneten und bei einer Abbremsung auf 90 MeV eine um den Faktor fünf höhere Transmission erreicht werden als es beispielsweise bei der Verwendung von ausschließlich Quadrupolen möglich ist. Im Vergleich zu Werten aus dem Stand der Technik sind somit deutlich höhere Transmissionswerte zu erreichen.

Bevorzugt weist das Strahlführungssystem in Richtung des Strahls geladener Teilchen gesehen nach der Energiekorrektureinheit eine Kollimatoreinheit auf. Hierdurch kann der Phasenraum nach der Energiekorrektureinheit eingegrenzt werden und die Strahlqualität am Behandlungsort verbessert werden. Vorzugsweise ist die Kollimatoreinheit unmittelbar nach der Energiekorrektureinheit angeordnet. Beispielsweise ist die Kollimatoreinheit als eine Blende ausgebildet. Eine Blende ist beispielsweise ein Materialblock mit einer oder mehreren Öffnungen. Beispielsweise weisen die Blenden jeweils eine kreisförmige und/oder eine entgegen der Richtung des Strahls geladener Teilchen gesehen konisch zulaufende Öffnung auf. Der Strahl geladener Teilchen durchläuft die Kollimatoreinheit bevorzugt in einem Vakuum.

Wie bereits ausgeführt ist die Kollimatoreinheit bevorzugt zwischen der Energiekorrektureinheit und einem unmittelbar dahinter vorgesehenen Solenoid-Magneten angeordnet.

Gemäß einer bevorzugten Ausgestaltung der erfindungsgemäßen Teilchenstrahl-Therapieanlage weist das Strahlführungssystem mindestens zwei Solenoid-Magnete in Richtung des Strahls geladener Teilchen gesehen hinter der Strahlerzeugungseinheit auf.

Durch das Vorsehen von mindestens zwei Solenoid-Magneten können die Strahleigenschaften bei hoher Kompaktheit der Teilchenstrahl-Therapieanlage weiterhin verbessert werden. Es hat sich insbesondere als vorteilhaft herausgestellt, dass das Vorsehen von genau zwei Solenoid-Magneten in dem Strahlführungssystem vorteilhaft ist. Dadurch können vorteilhafte Strahleigenschaften bei geringem Platzbedarf und geringem anlagentechnischen Aufwand erreicht werden.

Wie bereits ausgeführt, ist es besonders vorteilhaft, wenn ein erster Solenoid-Magnet zwischen der Strahlerzeugungseinheit und der Energiekorrektureinheit vorgesehen ist und ein zweiter Solenoid-Magnet hinter der Energiekorrektureinheit vorgesehen ist, insbesondere zwischen der Energiekorrektureinheit und einer Gantry.

Gemäß einer bevorzugten Ausgestaltung der erfindungsgemäßen Teilchenstrahl-Therapieanlage ist mindestens ein Solenoid-Magnet als eine im Wesentlichen geradlinige verlaufende Zylinderspule ausgebildet. Sind mehrere Solenoid-Magneten vorgesehen, sind bevorzugt alle Solenoid-Magneten jeweils als eine im Wesentlichen geradlinige verlaufende Zylinderspule ausgebildet. Hierdurch können einfache aufgebaute Solenoid-Magneten bereitgestellt werden, welche als Strahlformungseinheiten verwendet werden können. Beispielsweise ist die Zylinderspule einlagig ausgebildet. Insbesondere kann der Leiter (etwa ein Draht) der Zylinderspule einen schraubenförmigen oder helixförmigen Verlauf haben.

Gemäß einer bevorzugten Ausgestaltung der erfindungsgemäßen Teilchenstrahl-Therapieanlage verlaufen die Wicklungen des mindestens einen Solenoid-Magneten im Wesentlichen senkrecht zum Strahl geladener Teilchen. Sind mehrere Solenoid-Magneten vorgesehen, gilt dies bevorzugt für alle Solenoid-Magneten. Das heißt insbesondere, dass die Wicklungen des Leiters des jeweiligen Solenoid-Magneten jeweils im Wesentlichen in einer Ebene senkrecht zum Strahl geladener Teilchen verlaufen. Die Wicklungen sind also gegenüber dem Strahl geladener Teilchen nicht verkippt.

Gemäß einer bevorzugten Ausgestaltung der erfindungsgemäßen Teilchenstrahl-Therapieanlage erzeugt der mindestens eine Solenoid-Magnet zumindest abschnittsweise ein im Wesentlichen homogenes Magnetfeld. Unter einem homogenen Magnetfeld wird verstanden, dass die Feldstärke innerhalb des Solenoid-Magneten an jedem Ort gleich ist, also räumlich homogen ist. Das im Wesentlichen homogene Magnetfeld wird dabei in dem Bereich, welchen der Strahl geladener Teilchen durchquert und welcher insofern für die Formung des Strahl geladener Teilchen maßgeblich ist, erzeugt. Sind mehrere Solenoid-Magneten vorgesehen, gilt dies bevorzugt für alle Solenoid-Magneten.

Gemäß einer bevorzugten Ausgestaltung der erfindungsgemäßen Teilchenstrahl-Therapieanlage liegt das Magnetfeld mindestens eines Solenoid-Magneten im Bereich von 1 Tesla bis 10 Tesla, vorzugsweise im Bereich von 4 Tesla bis 8 Tesla und/oder das Magnetfeld mindestens eines Solenoid-Magneten im Bereich von 5 Tesla bis 20 Tesla, vorzugsweise im Bereich von 8 Tesla bis 15 Tesla. Derartige Magnetfeldstärken erlauben eine effektive Formung des Strahls geladener Teilchen und ermöglichen eine hohe Transmission bis zum Behandlungszentrum. Es ist besonders vorteilhaft, wenn mindestens zwei Solenoid-Magneten vorgesehen sind und die Magnetfelder der Solenoid-Magneten unterschiedlich sind. Beispielsweise liegt das Magnetfeld des einen Solenoid-Magneten (beispielsweise des in Strahlrichtung gesehen ersten Solenoid-Magneten, beispielsweise des zwischen Strahlerzeugungseinheit und Energiekorrektureinheit angeordneten Solenoid-Magneten) im Bereich von 5 Tesla bis 20 Tesla, vorzugsweise im Bereich von 8 Tesla bis 15 Tesla (beispielsweise bei etwa 11 - 11,5 Tesla) und das Magnetfeld des anderen Solenoid-Magneten (beispielsweise des in Strahlrichtung gesehen zweiten Solenoid-Magneten, beispielsweise des hinter der Energiekorrektureinheit angeordneten Solenoid-Magneten) im Bereich von 1 Tesla bis 10 Tesla, vorzugsweise im Bereich von 4 Tesla bis 8 Tesla (beispielsweise bei etwa 6 - 6,5 Tesla).

Gemäß einer bevorzugten Ausgestaltung der erfindungsgemäßen Teilchenstrahl-Therapieanlage ist die Teilchenstrahl-Therapieanlage derart eingerichtet, dass mindestens ein Solenoid-Magnet bei unterschiedlichen Energien der geladenen Teilchen des Strahls geladener Teilchen ein im Wesentlichen konstantes Magnetfeld erzeugt. Dies kann insbesondere den Vorteil haben, dass das Feld der Solenoid-Magneten (beispielsweise im Gegensatz zu Quadrupolmagenten) nicht oder nicht vollständig in Abhängigkeit der Energie der Energie der geladenen Teilchen gesteuert oder geregelt werden muss, was den Aufbau der Teilchenstrahl-Therapieanlage vereinfacht. Beispielsweise erzeugt der Solenoid-Magnet unabhängig von der Energie der geladenen Teilchen ein im Wesentlichen konstantes Magnetfeld. Beispielsweise erzeugt der Solenoid-Magnet für alle oder einen Teil der einstellbaren Teilchenenergien (beispielsweise von 70 MeV bis 200 MeV) ein im Wesentlichen konstantes Magnetfeld. Sind mehrere Solenoid-Magnete vorgesehen, erzeugen bevorzugt alle Solenoid-Magnete bei unterschiedlichen Energien der geladenen Teilchen des Strahls geladener Teilchen ein im Wesentlichen konstantes Magnetfeld. Dabei können die unterschiedlichen Solenoid-Magnete Magnetfelder unterschiedlicher Größe erzeugen.

Gemäß einer bevorzugten Ausgestaltung der erfindungsgemäßen Teilchenstrahl-Therapieanlage weist mindestens ein Solenoid-Magnet eine Eintrittsöffnung und/oder Austrittsöffnung zwischen 10 mm und 50 mm, vorzugsweise zwischen 20 mm und 40 mm auf und/oder mindestens ein Solenoid-Magnet eine Eintrittsöffnung und/oder Austrittsöffnung zwischen 30 mm und 70 mm, vorzugsweise zwischen 40 mm und 60 mm auf. Es hat sich gezeigt, dass bei derartigen Eintrittsöffnungen bzw. Austrittsöffnungen eine gewünschte Strahlformung erreicht werden kann. Beispielsweise sind mindestens zwei Solenoid-Magneten vorgesehen, welche unterschiedliche Eintrittsöffnungen und Austrittsöffnungen aufweisen. Beispielsweise weist ein erster Solenoid-Magnet (beispielsweise der in Strahlrichtung gesehen erste Solenoid-Magnet, beispielsweise der zwischen Strahlerzeugungseinheit und Energiekorrektureinheit angeordneten Solenoid-Magnet) eine Eintrittsöffnung und/oder Austrittsöffnung zwischen 10 mm und 50 mm, vorzugsweise zwischen 20 mm und 40 mm (beispielsweise etwa 30 mm) auf und der andere Solenoid-Magnet (beispielsweise der in Strahlrichtung gesehen zweite Solenoid-Magnet, beispielsweise der hinter der Energiekorrektureinheit angeordnete Solenoid-Magnet) eine Eintrittsöffnung und/oder Austrittsöffnung zwischen 30 mm und 70 mm, vorzugsweise zwischen 40 mm und 60 mm (beispielsweise etwa 52 mm) auf. Das Strahlführungssystem weist einen unbeweglichen Abschnitt und einen beweglichen, insbesondere rotierbaren Abschnitt auf.
Es hat sich gezeigt, dass durch das Vorsehen von mindestens einem supraleitendem Solenoid-Magneten als Strahlformungseinheit ein hoher Anteil der geladenen Teilchen in den rotierbaren Abschnitt geführt werden kann und der Strahl geladener Teilchen ein im Wesentlichen rotationssymmetrisches Profil um die Strahlachse am Eintritt in den rotierbaren Abschnitt ermöglicht.
Beispielsweise weist das Strahlführungssystem zur Realisierung des beweglichen Abschnitts ein bewegliches Traggestell, eine sogenannte Gantry, auf. Das Traggestell kann in vorteilhafter Weise, insbesondere um eine horizontale Achse, um bis zu 360° rotierbar sein, um den Behandlungsort aus möglichst vielen Winkeln bestrahlen zu können. Die Rotationsachse des Traggestells fällt insbesondere mit der ursprünglichen Achse des Strahls geladener Teilchen (d.h. insbesondere mit der Achse des Strahls geladener Teilchen vor einem Ablenken durch eine erste Strahlablenkeinheit) zusammen.
Der unbewegliche Abschnitt des Strahlführungssystems ist beispielsweise zwischen der Strahlerzeugungseinrichtung und dem beweglichen Abschnitt des Strahlführungssystems angeordnet.

Der bewegliche Abschnitt (insbesondere die Gantry) kann insbesondere weitere Elemente des Strahlführungssystems aufweisen, insbesondere einen oder mehrere (beispielsweise vier) Strahlablenkeinheiten, einen oder mehrere (beispielsweise sieben) Strahlformungseinheiten (beispielsweise in Form von Quadrupolmagneten), einen oder mehrere (beispielsweise zwei) Kollimatoreinheiten, einen oder mehrere Scanmagnete und/oder einen oder mehrere Strahlmonitore.

Grundsätzlich ist es auch denkbar den beweglichen Abschnitt des Strahlführungssystems frei von Kollimatoreinheiten auszugestalten. Dadurch kann das Strahlführungssystem kompakter ausgebildet werden.

Beispielsweise kann der bewegliche Abschnitt des Strahlführungssystems derart ausgebildet werden, dass der Strahl geladener Teilchen derart zum Behandlungsort geführt wird, dass der Strahl geladener Teilchen maximal 3 m von der ursprünglichen Achse des Strahls geladener Teilchen (beispielsweise wie der Strahl aus der Strahlerzeugungseinheit austritt) entfernt verläuft.

Gemäß einer bevorzugten Ausgestaltung der erfindungsgemäßen Teilchenstrahl-Therapieanlage ist der mindestens eine Solenoid-Magnet im unbeweglichen Abschnitt des Strahlführungssystems vorgesehen. Ist mehr als ein Solenoid-Magnet vorgesehen (beispielsweise zwei) sind bevorzugt alle Solenoid-Magnete im unbeweglichen Abschnitt des Strahlführungssystems vorgesehen.

Beispielsweise ist mindestens ein (vorzugsweise alle) Solenoid-Magnet(e) zwischen der Strahlerzeugungseinheit und dem rotierbarem Abschnitt des Strahlführungssystems angeordnet. Beispielsweise ist ein Solenoid-Magnet zwischen der Energiekorrektureinheit und dem beweglichen Abschnitt des Strahlführungssystems vorgesehen. Der bewegliche Abschnitt des Strahlführungssystems ist vorzugsweise frei von Solenoid-Magneten.

Die Energiekorrektureinheit (bevorzugt inklusive der dahinter angeordneten Kollimatoreinheit) ist vorzugsweise im unbeweglichen Abschnitt des Strahlführungssystems vorgesehen.

Gemäß einer bevorzugten Ausgestaltung der erfindungsgemäßen Teilchenstrahl-Therapieanlage, weist das Strahlführungssystem mindestens eine magnetische Strahlablenkeinheit, insbesondere im beweglichen Abschnitt des Strahlführungssystems, auf. Sind mehrere magnetische Strahlablenkeinheiten vorgesehen, sind bevorzugt alle im beweglichen Abschnitt des Strahlführungssystems vorgesehen. Durch die mindestens eine Strahlablenkeinheit kann der Strahl geladener Teilchen vorteilhaft und bedarfsgerecht von der Strahlerzeugungseinheit zum Behandlungszentrum geführt werden. Dabei kann die magnetische Strahlablenkeinheit eine Eintrittsseite zum Eintreten des Strahls geladener Teilchen unter einer Eintrittsrichtung in die magnetische Strahlablenkeinheit und eine Austrittsseite zum Austreten des Strahls geladener Teilchen unter einer Austrittrichtung aus der magnetischen Strahlablenkeinheit aufweisen.

Die mindesten eine magnetische Strahlablenkeinheit ist bevorzugt ein Dipolmagnet. Durch die Ausgestaltung der magnetischen Strahlablenkeinheit als Dipolmagnet kann auf einfache Weise ein im Wesentlichen homogenes Magnetfeld zur Ablenkung des Strahls geladener Teilchen bereitgestellt werden. Zudem können Dipolmagnete vergleichsweise einfach gefertigt werden. Der Dipolmagnet ist beispielsweise ein Elektromagnet. Beispielsweise weist der Dipolmagnet einen Eisenkern, beispielsweise ein Eisenjoch, auf. Der Eisenkern kann beispielsweise Eisenplatten aufweisen. Beispielsweise ist der Eisenkern durch aufeinander gestapelte Eisenplatten hergestellt. Dies ermöglicht eine einfache Fertigung des Dipolmagneten. Der Dipolmagnet kann beispielsweise in Richtung des Strahls geladener Teilchen gesehen einen Abstand der Eintrittsseite und der Austrittsseite von 0,5 bis 2 m aufweisen, bevorzugt etwa 1 m. Es hat sich gezeigt, dass der Einsatz von Dipolmagneten in dem Strahlführungssystem gute Strahleigenschaften ermöglicht.

Bevorzugt weist das Strahlführungssystem mehrere, beispielsweise zwei, drei oder insbesondere vier magnetische Strahlablenkeinheiten auf. Sind mehrere magnetische Strahlablenkeinheiten vorgesehen kann ein komplexer Verlauf des Strahls geladener Teilchen in einem kompakten Strahlführungssystem erreicht werden.

Bevorzugt sind die mehreren Strahlablenkeinheiten derart in dem Strahlführungssystem vorgesehen, dass der Strahl geladener Teilchen (zu einem bestimmten Zeitpunkt) im Wesentlichen in einer Ebene verläuft. Beispielsweise kann durch die Rotation des beweglichen Abschnitts diese Eben gedreht werden.

Beispielsweise ist das Strahlführungssystem derart eingerichtet, dass der Strahl geladener Teilchen durch die erste Strahlablenkeinheit von der ursprünglichen Achse des Strahls geladener Teilchen weggelenkt wird und schräg zu der ursprünglichen Achse verläuft. Die ursprüngliche Achse des Strahls geladener Teilchen ist beispielsweise die Achse, entlang der Strahl geladener Teilchen aus der Strahlerzeugungseinheit austritt und/oder entlang der er sich vor der ersten Strahlablenkeinheit bewegt. Beispielsweise ist das Strahlführungssystem derart eingerichtet, dass der Strahl geladener Teilchen durch die weiteren magnetischen Strahlablenkeinheiten (beispielsweise eine zweite, dritte und vierte magnetische Strahlablenkeinheit) wieder zu der ursprünglichen Achse des Strahls geladener Teilchen hinlenkt wird. Beispielsweise ist das Strahlführungssystem derart eingerichtet, dass der Strahl geladener Teilchen nach der zweiten Strahlablenkeinheit parallel zu der ursprünglichen Achse des Strahls geladener Teilchen verläuft. Beispielsweise ist das Strahlführungssystem derart eingerichtet, dass der Strahl geladener Teilchen nach der letzten (beispielsweise der vierten) Strahlablenkeinheit quer, insbesondere im Wesentlichen senkrecht, zu der ursprünglichen Achse des Strahls geladener Teilchen verläuft und vorzugsweise die ursprüngliche Achse kreuzt.

Vorzugsweise ist zumindest ein Teil der mehreren magnetischen Strahlablenkeinheiten identisch ausgebildet. Hierdurch kann der Fertigungsaufwand für die magnetischen Strahlablenkeinheiten reduziert werden. Beispielsweise sind alle bis auf eine magnetische Strahlablenkeinheit (beispielsweise die letzte magnetische Strahlablenkeinheit vor dem Behandlungsort) identisch ausgebildet. Beispielsweise ist die vierte Strahlablenkeinheit abweichend ausgebildet. Durch eine abweichende Ausbildung insbesondere der letzten magnetischen Strahlablenkeinheit kann eine Anpassung an die Bedingungen im Strahlverlauf erfolgen. Beispielsweise weist die vierte Strahlablenkeinheit eine im Vergleich zu den anderen magnetischen Strahlablenkeinheiten vergrößerte Eintrittsöffnung und/oder Austrittsöffnung auf, um eine Bestrahlung eines ausreichenden Volumens am Behandlungsort zur Verfügung zu stellen.

Beispielsweise sind die in Richtung des Strahls geladener Teilchen gesehen zweite und dritte magnetische Strahlablenkeinheit 0,5 m bis 2 m, vorzugsweise 1 m bis 1,5 m voneinander entfernt. Beispielsweise ist die in Richtung des Strahls geladener Teilchen gesehen letzte magnetische Strahlablenkeinheit höchstens 1,5 m, vorzugsweise höchstens 0,99 m vom Behandlungsort entfernt. Unter dem Abstand von zwei magnetischen Strahlablenkeinheiten wird insbesondere der Abstand von dem Austrittspunkt des Strahls geladener Teilchen aus der einen magnetischen Strahlablenkeinheit bis zum Eintrittspunkt des Strahls geladener Teilchen in die andere magnetische Strahlablenkeinheit verstanden.

Der Strahl geladener Teilchen kann mit dem Strahlführungssystem beispielsweise auf einer Strecke von weniger als 10 m, insbesondere weniger als 8 m von der Richtung entlang der ursprünglichen Achse des Strahl geladener Teilchen in eine Richtung senkrecht hierzu und die ursprüngliche Achse kreuzend abgelenkt werden.

Bevorzugt ist die Eintrittsseite der (zumindest einen) magnetischen Strahlablenkeinheit zumindest abschnittsweise im Wesentlichen parallel zu der Austrittsseite ausgebildet.

Es hat sich gezeigt, dass, wenn ein Strahl geladener Teilchen von einer Eintrittsrichtung in eine von der Eintrittsrichtung abweichende Austrittsrichtung abgelenkt werden soll, durch eine derartige Ausbildung der Strahlablenkeinheit die Strahleigenschaften und die Kompaktheit des Strahlführungssystems weiterhin verbessert werden können. Dies wird unter anderem darauf zurückgeführt, dass derartige magnetische Strahlablenkeinheiten nicht nur ein Ablenken des Strahls geladener Teilchen, sondern zudem auch eine Fokussierung des Strahls geladener Teilchen ähnlich einer Strahlformungseinheit erreichen können. Denn die magnetoptischen Eigenschaften einer magnetischen Strahlablenkeinheit (zum Beispiel eines Dipolmagneten) werden wesentlich durch den Winkel des eintreffenden Strahls geladener Teilchen gegenüber der Eintritts- und Austrittskante bestimmt. Dadurch sind im gesamten Strahlführungssystem weniger Strahlformungseinheiten notwendig. Zudem kann eine erhöhte Transmission des Strahls geladener Teilchen durch das Strahlführungssystem erreicht werden. Dies führt dazu, dass insgesamt eine kompaktere Teilchenstrahl-Therapieanlage mit verbesserten Strahleigenschaften bereitgestellt werden kann. Zusätzlich ist auch die Fertigung von Strahlablenkeinheiten, bei denen die Eintrittsseite zumindest abschnittsweise im Wesentlichen parallel zu der Austrittsseite ausgebildet ist, einfacher durchzuführen, was zusätzlich den Herstellungsaufwand reduziert.

Beispielsweise ist die Eintrittsseite und/oder die Austrittsseite der magnetischen Strahlablenkeinheit zumindest abschnittsweise flächig ausgebildet. In dem Fall sind insbesondere die flächigen Abschnitte der Eintrittsseite und der Austrittsseite zumindest abschnittsweise im Wesentlichen parallel zueinander ausgebildet. Die Eintrittsseite kann insbesondere eine Stirnfläche, beispielsweise eine Vorderseite der Strahlablenkeinheit sein, welche beispielsweise eine Eintrittsöffnung zum Eintreten des Strahls geladener Teilchen aufweist. Die Austrittsseite kann insbesondere eine weitere Stirnfläche, beispielsweise eine der Eintrittsseite gegenüberliegende Stirnfläche, beispielsweise eine Rückseite der Strahlablenkeinheit sein, welche beispielsweise eine Austrittsöffnung zum Austreten des Strahls geladener Teilchen aufweist.

Bevorzugt sind die gesamte Eintrittsseite und die gesamte Austrittsseite im Wesentlich parallel zu einander. Unter im Wesentlichen parallel wird verstanden, dass die Eintrittsseite und die Austrittsseite beispielsweise einen Winkel von weniger als 5°, vorzugsweise einen Winkel von weniger als 3°, besonders bevorzugt weniger als 1° einschließen.

Die magnetische Strahlablenkeinheit ist zur Ablenkung des Strahls geladener Teilchen beispielsweise derart in dem Strahlführungssystem vorgesehen, dass der Strahl von der Eintrittsrichtung in die gewünschte Austrittsrichtung abgelenkt wird.

Die magnetische Strahleinheit ist bevorzugt zu einer zeitlich konstanten Ablenkung des Strahls geladener Teilchen ausgebildet. Das heißt, dass der Strahl geladener Teilchen beispielsweise dauerhaft um 45° abgelenkt wird.

Die Strahlablenkeinheit kann beispielsweise derart ansteuerbar sein, dass eine gewünschte Ablenkung beispielsweise für unterschiedliche Energien der geladenen Teilchen des Strahls der geladenen Teilchen möglich ist.

Die magnetische Strahlablenkeinheit kann derart zur Ablenkung des Strahls geladener Teilchen in dem Strahlführungssystem vorgesehen sein, dass die Eintrittsseite schräg zur Eintrittsrichtung des Strahls geladener Teilchen und/oder die Austrittsseite schräg zur Austrittsrichtung des Strahls geladener Teilchen liegt.

Der Strahl geladener Teilchen kann in dem Strahlführungssystem insbesondere unter einer definierten Eintrittsrichtung in die Strahlablenkeinheit eintreten und unter einer definierten Austrittsrichtung aus der Strahlablenkeinheit austreten. Dadurch, dass die Eintrittsseite schräg zur Eintrittsrichtung des Strahls geladener Teilchen bzw. die Austrittsseite schräg zur Austrittsrichtung des Strahls geladener Teilchen liegt, kann die Strahlablenkeinheit insbesondere symmetrisch in dem Strahl geladener Teilchen vorgesehen werden, was die Strahleigenschaften weiterhin verbessert. Unter einer schrägen Anordnung wird dabei insbesondere verstanden, dass eine Seite insbesondere nicht senkrecht auf und/oder nicht parallel zu der Eintritts- oder Austrittsrichtung steht. Beispielsweise verlaufen die Eintritts- und/oder die Austrittsseite im Wesentlichen parallel zu der Mittelsenkrechten des Winkels, der zwischen der Eintrittsrichtung und der Austrittsrichtung des Strahls geladener Teilchen gebildet wird. Beispielsweise ist der Winkel zwischen der Eintrittsseite und der Eintrittsrichtung und zwischen der Austrittsseite und der Austrittsrichtung gleich.

Die magnetische Strahlablenkeinheit kann derart zur Ablenkung des Strahls geladener Teilchen in dem Strahlführungssystem vorgesehen sein, dass die Eintrittsrichtung und die Austrittsrichtung in einem Winkel von 30° bis 60°, vorzugsweise von 40° bis 50°, insbesondere im Wesentlichen 45°, zueinander stehen.

Der Strahl geladener Teilchen wird also beispielsweise um 30° bis 60°, vorzugsweise um 40° bis 50°, insbesondere um im Wesentlichen 45°, abgelenkt. Es hat sich gezeigt, dass eine Ablenkung des Strahls geladener Teilchen bei diesen Winkeln mit guten Strahleigenschaften erfolgen und gleichzeitig ein kompaktes Strahlführungssystem bereitgestellt werden kann.

Wie bereits ausgeführt, können insbesondere mehrere derartige magnetische Strahlablenkeinheiten vorgesehen sein.

Gemäß einer bevorzugten Ausgestaltung der erfindungsgemäßen Teilchenstrahl-Therapieanlage ist mindestens ein Solenoid-Magnet in Richtung des Strahls geladener Teilchen gesehen vor der mindestens einen magnetischen Strahlablenkeinheit angeordnet. Durch das Anordnen des Solenoid-Magneten vor einer magnetischen Strahlablenkeinheit wird der Strahl geladener Teilchen vor der Ablenkung fokussiert. Dies kann zur Erhöhung der Transmission bis zum Behandlungszentrum beitragen. Insbesondere kann mindestens ein Solenoid-Magnet vor der ersten magnetischen Strahlablenkeinheit vorgesehen sein, sodass die Strahleigenschaften vor einer ersten Ablenkung des Strahls geladener Teilchen optimiert werden können.

Gemäß einer bevorzugten Ausgestaltung der erfindungsgemäßen Teilchenstrahl-Therapieanlage weist das Strahlführungssystem mindestens eine zusätzliche magnetische Strahlformungseinheit, insbesondere in Form eines Quadrupolmagneten, insbesondere im beweglichen Abschnitt des Strahlführungssystems, auf.

Durch das Vorsehen von magnetischen Strahlformungseinheiten, welche zusätzlich zu dem mindestens einen Solenoid-Magneten vorgesehen sind, insbesondere in Form von Quadrupolmagneten, kann die Strahleigenschaft weiter verbessert werden und der Strahl geladener Teilchen effizient fokussiert werden. Es hat sich gezeigt, dass in dem Strahlführungssystem vergleichsweise wenige zusätzliche Strahlformungseinheiten notwendig sind, um gute Strahleigenschaften zu erzielen, was ein kompaktes Strahlführungssystem erlaubt. Grundsätzlich können aber auch andersartige zusätzliche Strahlformungseinheiten vorgesehen sein. Beispielsweise können Strahlformungseinheiten in Form von Sextupolmagnete vorgesehen sein.

Das Magnetfeld der zusätzlichen magnetischen Strahlformungseinheit(en) (beispielsweise im Falle von Quadrupolmagneten) wird beispielsweise bereichsweise (etwa für Energien von höchstens 120 MeV) in Abhängigkeit des mittleren Impulses der Teilchen des Strahls geladener Teilchen (nach der Energiekorrektureinheit) skaliert. Für größere Energien kann eine abweichende Skalierung vorgesehen sein.

Quadrupolmagnete können aufgrund ihres Aufbaus einen Strahl geladener Teilchen nur in einer Richtung transversal zum Strahl geladener Teilchen fokussieren. Insofern ist es vorteilhaft mindestens zwei Quadrupolmagnete vorzusehen, um eine Strahlformung in beide Richtungen (d.h. in der Ebene) transversal zum Strahl geladener Teilchen zu erreichen.

Beispielsweise sind mindestens fünf und/oder höchstens zehn, vorzugsweise sieben zusätzliche Strahlformungseinheiten in dem Strahlführungssystem vorgesehen. Beispielsweise sind mindestens vier und/oder höchstens sechs, vorzugsweise fünf zusätzliche Strahlformungseinheiten zwischen einer (in Richtung des Strahls geladener Teilchen gesehen) ersten und einer zweiten magnetischen Strahlablenkeinheit vorgesehen. Beispielsweise sind zwei zusätzliche Strahlformungseinheiten zwischen der zweiten und der dritten magnetischen Strahlablenkeinheit vorgesehen. Es können jedoch auch weitere zusätzliche Strahlformungseinheiten vorgesehen sein. Beispielsweise sind die zusätzlichen Strahlformungseinheiten zumindest teilweise, vorzugsweise alle gleich dimensioniert. Dies verringert den Herstellungsaufwand des Strahlführungssystems.

Bevorzugt weist das Strahlführungssystem in Richtung des Strahls geladener Teilchen gesehen vor der ersten magnetischen Strahlablenkeinheit eine Kollimatoreinheit auf.

Hierdurch können die Strahleigenschaften bei geringem zusätzlichem Platzbedarf verbessert werden. Durch die Kollimatoreinheit kann der Phasenraum des Strahls geladener Teilchen des hinter der Kollimatoreinheit weiter transportierten Strahls eingegrenzt werden.

Beispielsweise ist die Kollimatoreinheit als Blende ausgebildet, beispielsweise als Materialblock mit einer oder mehreren Öffnungen. Beispielsweise weist die Kollimatoreinheit eine eckige, beispielsweise eine rechteckige Öffnung auf. Vorzugsweise lässt sich die Öffnung in beide Richtungen transversal zum Strahl geladener Teilchen verändern. Damit kann eine flexible Anpassung der Strahleigenschaften erfolgen.

Bevorzugt weist das Strahlführungssystem mindestens zwei magnetische Strahlablenkeinheiten und zwischen zwei der mindestens zwei magnetischen Strahlablenkeinheiten eine Kollimatoreinheit auf.

Es hat sich gezeigt, dass insbesondere zwischen zwei Strahlablenkeinheiten eine große Impulsdispersion herrschen kann. Durch das Vorsehen einer Kollimatoreinheit können die Strahleigenschaften bei geringem zusätzlichem Platzbedarf weiterhin verbessert werden. Es hat sich gezeigt, dass insbesondere zwischen der zweiten und dritten Strahlablenkeinheit eine vergleichsweise große Impulsdispersion herrscht, sodass durch das Vorsehen einer Kollimatoreinheit die Strahleigenschaften besonders verbessert werden können. Die Kollimatoreinheit kann daher insbesondere dazu verwendet werden, eine Impulsselektion für den Strahl geladener Teilchen am Behandlungsort zu erreichen. Insbesondere kann eine Formgebung für den Strahlfleck am Behandlungsort erfolgen. Es kann zudem erreicht werden, dass die Verluste hinter dem Kollimator bis zum Behandlungsort kleiner 1% betragen, obwohl im Strahlführungssystem in Strahlrichtung gesehen weitere, beispielsweise noch zwei Strahlablenkeinheiten angeordnet sind.

Bevorzugt weist das Strahlführungssystem vor der in Richtung des Strahls geladener Teilchen gesehen ersten magnetischen Strahlablenkeinheit eine Driftstrecke auf, welche frei von magnetischen Strahlablenkeinheiten und/oder magnetischen Strahlformungseinheiten ist.

Durch die Driftstrecke kann vorteilhaft ein Bereich zur Verfügung gestellt werden, welcher beispielsweise eine oder mehrere Messeinrichtungen (insbesondere Strahlmonitore) zur Strahlkontrolle aufnehmen kann. Ebenfalls kann dieser Bereich zur Abschirmung (beispielsweise mittels einer Betonabschirmung) des Behandlungsortes von der Strahlerzeugungseinheit dienen. Beispielsweise beträgt die Länge der Driftstrecke mindestens 1 m und/oder höchstens 2 m. Die Driftstrecke ist bevorzugt zumindest teilweise im Vakuum vorgesehen, beispielsweise in einem Vakuumrohr von bevorzugt wenigen Zentimetern Durchmesser. Bevorzugt ist die Driftstrecke in Richtung des Strahls geladener Teilchen gesehen hinter einem Solenoid-Magneten angeordnet. Bevorzugt ist die Driftstrecke frei von jeglichen magnetischen Komponenten. Die Driftstecke ist vorzugsweise im unbeweglichen Abschnitt des Strahlführungssystems vorgesehen.

Die Teilchenstrahl-Therapieanlage und insbesondere das Strahlführungssystem kann noch weitere hier nicht erwähnte Einheiten aufweisen. Beispielsweise kann ein Scanmagnet im Strahlführungssystem vorgesehen sein. Beispielsweise ist der Scanmagnet zwischen zwei Strahlablenkeinheiten vorgesehen. Vorzugsweise ist der Scanmagnet zwischen der letzten und der vorletzten (beispielsweise zwischen der dritten und der vierten) Strahlablenkeinheit vorgesehen, da der Scan-Magnet in dieser Position vorteilhaft einen großen Scan-Bereich am Behandlungsort ermöglicht.

Als weiteres Beispiel einer weiteren Einheit kann das Strahlführungssystem einen oder mehrere Strahlmonitore aufweisen. Durch Strahlmonitore können die Strahleigenschaften, beispielsweise die Strahlposition und der Teilchenimpuls des Strahls geladener Teilchen insbesondere an unterschiedlichen Stellen vermessen werden.

Gemäß einem zweiten Aspekt der vorliegenden Erfindung wird die eingangs genannte Aufgabe auch durch ein Verfahren, ausgeführt mit einer erfindungsgemäßen Teilchenstrahl-Therapieanlage, gelöst, umfassend die Schritte Erzeugen eines Strahls geladener Teilchen, insbesondere Ionen, vorzugsweise Protonen, mit der Strahlerzeugungseinheit, und Führen des Strahls geladener Teilchen mittels des Strahlführungssystems.

Bezüglich der Vorteile und weiteren Ausgestaltungen des Verfahrens wird auf den vorherigen Aspekt und dessen Ausgestaltungen verwiesen.

Insbesondere soll durch die vorherige und folgende Beschreibung von Mitteln zur Durchführung eines Verfahrensschrittes der entsprechende Verfahrensschritt offenbart sein. Ebenfalls sollen durch die Offenbarung von Verfahrensschritten auch entsprechende Mittel oder Einrichtungen zur Durchführung der Verfahrensschritte offenbart sein.

Die oben beschriebenen Ausführungsbeispiele und beispielhaften Ausgestaltungen aller Aspekte der vorliegenden Erfindung sollen auch in allen Kombinationen miteinander offenbart verstanden werden.

Weitere vorteilhafte beispielhafte Ausgestaltungen der unterschiedlichen Aspekte sind der folgenden detaillierten Beschreibung einiger beispielhafter Ausführungsformen der Aspekte, insbesondere in Verbindung mit den Figuren zu entnehmen. Die der Anmeldung beiliegenden Figuren sollen jedoch nur dem Zwecke der Verdeutlichung, nicht aber zur Bestimmung des Schutzbereiches der Erfindung dienen. Die beiliegenden Zeichnungen sind nicht notwendigerweise maßstabsgetreu und sollen lediglich das allgemeine Konzept der vorliegenden Aspekte beispielhaft widerspiegeln. Insbesondere sollen Merkmale, die in den Figuren enthalten sind, keineswegs als notwendiger Bestandteil der vorliegenden Erfindung erachtet werden.

Die Erfindung ist in den Ansprüchen definiert; weitere Ausführungsformen sind lediglich beispielhaft. Es zeigen:
- Fig. 1: eine schematische Schnittansicht eines Ausführungsbeispiels einer Teilchenstrahl-Therapieanlage mit einem Ausführungsbeispiel eines Strahlführungssystems gemäß der vorliegenden Erfindung;
- Fig. 2: eine schematische Schnittansicht eines weiteren Ausführungsbeispiels des beweglichen Abschnitts eines Strahlführungssystems;
- Fig. 3: eine schematische Schnittansicht eines Ausführungsbeispiels einer Energiekorrektureinheit.

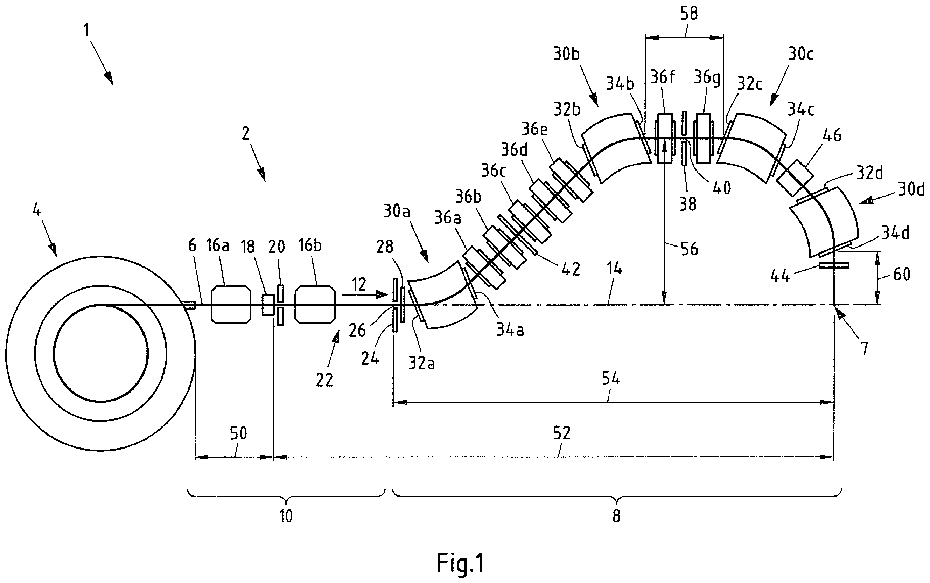

Fig. 1 zeigt eine schematische Schnittansicht eines Ausführungsbeispiels einer Teilchenstrahl-Therapieanlage 1 mit einem Ausführungsbeispiel eines Strahlführungssystems 2. Die Teilchenstrahl-Therapieanlage umfasst eine Strahlerzeugungseinheit 4 zur Erzeugung eines Strahls geladener Teilchen 6, welche insbesondere Protonen sein können. Der Strahl geladener Teilchen 6 wird durch das Strahlführungssystem 2 an einen Behandlungsort 7 geführt. Das Strahlführungssystem 2 weist einen beweglichen, in diesem Fall einen um 360° rotierbaren Abschnitt 8 und einen unbeweglichen Abschnitt 10 auf. Der rotierbare Abschnitt 8 kann beispielsweise durch ein Traggestell in Form einer Gantry (nicht dargestellt) realisiert sein.

Die Strahlerzeugungseinheit 4 ist in diesem Fall ein Zyklotron, das heißt eine Beschleunigereinrichtung, welche den Strahl geladener Teilchen 6 mit konstanter Energie erzeugt. Die Strahlerzeugungseinheit 4 emittiert geladene Teilchen mit einer konstanten kinetischen Energie, beispielsweise 210 MeV, 215 MeV oder 250 MeV. Es hat sich gezeigt, dass mittels geladener Teilchen mit einer kinetischen Energie von etwa 207MeV etwa 95%, mittels geladener Teilchen mit einer kinetischen Energie von etwa 198 MeV noch etwa 90% der zu behandelnden Patienten behandelt werden können.

Der Strahl geladener Teilchen verläuft zunächst in Richtung des Pfeils 12 entlang einer ursprünglichen Achse 14 des Strahls geladener Teilchen 6.

In Richtung 12 des Strahls geladener Teilchen 6 gesehen hinter der Strahlerzeugungseinheit 4 ist ein erster supraleitender Solenoid-Magnet 16a als Strahlformungseinheit vorgesehen. In diesem Fall ist der Solenoid-Magnet 16a unmittelbar hinter der Strahlerzeugungseinheit vorgesehen. Ebenfalls ist der Solenoid-Magnet 16a zwischen der Strahlerzeugungseinheit 4 und einer Energiekorrektureinheit 18 vorgesehen. Das Magnetfeld des Solenoid-Magneten 16a beträgt in diesem Fall etwa 11.2 Tesla. Die Eintrittsöffnung und die Austrittsöffnung des Solenoid-Magneten 16a betragen in diesem Fall etwa 30 mm. In Abhängigkeit der Auslegung der Teilchenstrahl-Therapieanlage und des Strahlführungssystems können jedoch auch andere Magnetfeldstärken und Öffnungsgrößen möglich bzw. notwendig sein.

Durch den Solenoid-Magneten 16a kann eine verbesserte Abbildung des von der Strahlerzeugungseinheit emittierten Phasenraums auf die dahinter angeordnete Energiekorrektureinheit 18 ermöglicht werden, sodass die Strahlqualität und Transmission des Strahls geladener Teilchen 6 bis zu dem Behandlungsort 7 erhöht werden kann.

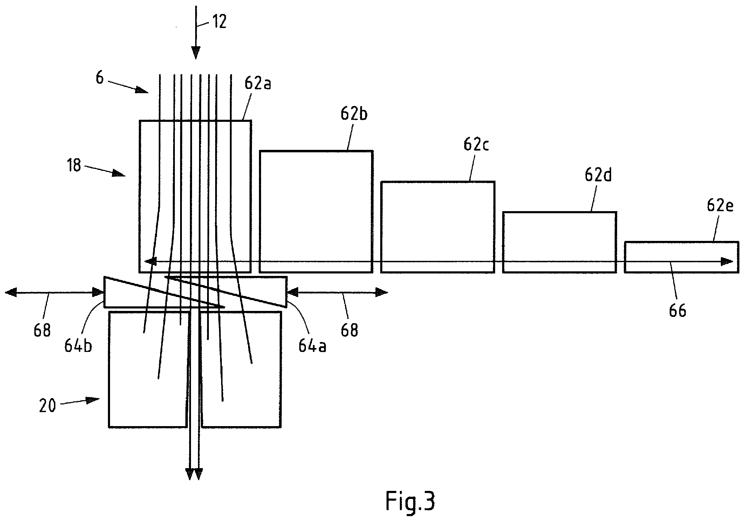

Der Strahl geladener Teilchen 6 wird anschließend durch die Energiekorrektureinheit 18 geführt. Durch die Energiekorrektureinheit 18 kann eine Einstellung der Energie der geladenen Teilchen des Strahls geladener Teilchen 6 erfolgen. Die Energiekorrektureinheit wird in Zusammenhang mit Fig. 3 näher beschrieben.

Das Strahlführungssystem 2 weist zudem in Richtung 12 des Strahls geladener Teilchen 6 gesehen unmittelbar nach der Energiekorrektureinheit 18 eine Kollimatoreinheit 20 auf, welche der Strahl der geladenen Teilchen 6 anschließend durchläuft. Die Kollimatoreinheit umfasst eine Blende, welche als Materialblock mit jeweils einer kreisförmigen und entgegen der Richtung 12 des Strahls geladener Teilchen 6 gesehen konisch zulaufenden Öffnung ausgebildet ist.

Weiter in Richtung 12 des Strahls geladener Teilchen 6 gesehen hinter der Energiekorrektureinheit 18 und der Kollimatoreinheit 20 ist ein zweiter supraleitender Solenoid-Magnet 16b als Strahlformungseinheit vorgesehen. Der Solenoid-Magnet 16b ist zwischen der Energiekorrektureinheit 18 und dem beweglichen Abschnitt 8 des Strahlführungssystems 2 und insbesondere vor einer ersten magnetischen Strahlablenkeinheit 30a vorgesehen. Das Magnetfeld des Solenoid-Magneten 16b beträgt in diesem Fall etwa 6.16 Tesla. Die Eintrittsöffnung und die Austrittsöffnung des Solenoid-Magneten 16b betragen in diesem Fall etwa 52 mm. In Abhängigkeit der Auslegung der Teilchenstrahl-Therapieanlage und des Strahlführungssystems können jedoch auch andere Magnetfeldstärken und Öffnungsgrößen möglich bzw. notwendig sein.

Durch den Solenoid-Magneten 16b kann eine verbesserte Abbildung des von der Energiekorrektureinheit 18 emittierten Phasenraums auf eine dahinter angeordnete Kollimatoreinheit 24 (und damit in den beweglichen Abschnitt 8 des Strahlführungssystems 2) ermöglicht werden, sodass die Strahlqualität und Transmission des Strahls geladener Teilchen 6 bis zu dem Behandlungsort 7 erhöht werden kann.

Sowohl der erste Solenoid-Magnet 16a als auch der zweite Solenoid-Magnet 16b sind beispielsweise als eine im Wesentlichen geradlinig verlaufende Zylinderspule ausgebildet und erzeugen im Bereich des Strahls geladener Teilchen 6 ein im Wesentlichen homogenes Magnetfeld. Vorteilhaft ist die Teilchenstrahl-Therapieanlage 1 derart eingerichtet, dass die Solenoid-Magnete 16a, 16b unabhängig von der Energie der geladenen Teilchen des Strahls geladener Teilchen 6 ein im Wesentlichen konstantes Magnetfeld erzeugen, sodass eine komplexe Energieabhängige Ansteuerung des Solenoid-Magnete 16a, 16b entfallen kann.

Das Strahlführungssystem 2 weist nach dem zweiten Solenoid-Magneten 16b in Richtung 12 des Strahls geladener Teilchen 6 gesehen eine Driftstrecke 22 auf. Die Driftstrecke 22 ist frei von magnetischen Einheiten, wie magnetische Strahlablenkeinheiten oder magnetischen Strahlformungseinheiten. Im Bereich der Driftstrecke 22 kann eine Abschirmung, etwa eine Betonabschirmung (nicht dargestellt) vorgesehen sein. Zudem können in dem Bereich der Driftstrecke 22 Messeinrichtungen wie Strahlmonitore (nicht dargestellt) vorgesehen sein.

Der Strahl geladener Teilchen 6 kann den Abschnitt der Energiekorrektureinheit 18, der Kollimatoreinheit 20 und/oder der Driftstrecke 22 des Strahlführungssystems 2 in einem Vakuum durchlaufen, was die Strahleigenschaften und die Transmission in diesem Abschnitt verbessert.

Die bisher beschriebenen Elemente des Strahlführungssystems 2 sind in dem unbeweglichen Abschnitt 10 des Strahlführungssystems 2 angeordnet. Um den Behandlungsort 7 aus möglichst vielen Winkeln bestrahlen zu können, ist der rotierbare Abschnitt 8 vorgesehen. Die Rotationsachse des Traggestells (nicht dargestellt) fällt mit der ursprünglichen Achse 14 des Strahls geladener Teilchen 6 zusammen.

In dem rotierbaren Abschnitt 8 weist das Strahlführungssystem zunächst eine Kollimatoreinheit 24 auf. Durch die Kollimatoreinheit 24 kann der Phasenraum des Strahls geladener Teilchen definiert werden. Die Kollimatoreinheit 24 ist hier als Blende ausgebildet, in diesem Fall als Materialblock mit einer rechteckigen Öffnung 26. Die Geometrie der Öffnung 26 lässt sich in beide Richtungen transversal zum Strahl geladener Teilchen 6 verändern, sodass eine flexible Anpassung der Strahleigenschaften erfolgen kann.

Anschließend ist ein optionaler Strahlmonitor 28 vorgesehen, um die Strahleigenschaften zu überprüfen. Der Strahlmonitor kann zusätzlich oder alternativ auch an anderen Stellen des Strahlführungssystems 2 vorgesehen werden.

Anschließend weist das Strahlführungssystem 2 zur Führung des Strahls geladener Teilchen 6 mehrere, in diesem Fall vier als magnetische Dipole ausgebildete magnetische Strahlablenkeinheiten 30a, 30b, 30c, 30d auf. Die magnetischen Strahlablenkeinheiten 30a, 30b, 30c, 30d weisen jeweils eine Eintrittsseite 32a, 32b, 32c, 32d zum Eintreten des Strahls geladener Teilchen 6 unter einer Eintrittsrichtung in die jeweilige magnetische Strahlablenkeinheit auf. Die magnetische Strahlablenkeinheiten 30a, 30b, 30c, 30d weisen zudem jeweils eine Austrittsseite 34a, 34b, 34c, 34d zum Austreten des Strahls geladener Teilchen 6 unter einer Austrittrichtung aus der magnetische Strahlablenkeinheit 30a, 30b, 30c, 30d auf. Die Eintrittsseiten 32a, 32b, 32c, 32d sind jeweils parallel zu der jeweiligen Austrittsseite 34a, 34b, 34c, 34d ausgebildet. Dabei sind die magnetischen Strahlablenkeinheit 30a, 30b, 30c, 30d jeweils derart zur Ablenkung des Strahls geladener Teilchen 6 in dem Strahlführungssystem 2 vorgesehen, dass die jeweiligen Eintrittsseite 32a, 32b, 32c, 32d schräg zur jeweiligen Eintrittsrichtung des Strahls geladener Teilchen 6 und die jeweiligen Austrittsseite 34a, 34b, 34c, 34d schräg zur jeweiligen Austrittsrichtung des Strahls geladener Teilchen 6 liegt. Dabei sind die magnetischen Strahlablenkeinheiten 30a, 30b, 30c, 30d in diesem Fall derart zur Ablenkung des Strahls geladener Teilchen 6 in dem Strahlführungssystem 2 vorgesehen, dass die Eintrittsrichtung und die Austrittsrichtung bei jeder Strahlablenkeinheit 30a, 30b, 30c, 30d in einem Winkel von 45° zueinander stehen. Die Strahlablenkeinheiten 30a, 30b, 30c, 30d sind dabei symmetrisch in dem Strahl geladener Teilchen positioniert, das heißt der Winkel zwischen Eintrittsrichtung und Eintrittsseite 32a, 32b, 32c, 32d und der Winkel zwischen Austrittsrichtung und Austrittsseite 34a, 34b, 34c, 34d sind bei den einzelnen Strahlablenkeinheiten 30a, 30b, 30c, 30d jeweils identisch.

Der Strahl geladener Teilchen 6 wird durch die erste Strahlablenkeinheit 30a von der ursprünglichen Achse 14 des Strahls geladener Teilchen weggelenkt. Der Strahl geladener Teilchen 6 wird durch die zweite Strahlablenkeinheit 30b wieder zu der ursprünglichen Achse 14 hingelenkt und verläuft dann parallel zu der ursprünglichen Achse 14 des Strahls geladener Teilchen 6. Anschließend wird der Strahl geladener Teilchen 6 durch die dritte und die vierte Strahlablenkeinheit 30c, 30d ebenfalls wieder zu der ursprünglichen Achse 14 hingelenkt, sodass der Strahl geladener Teilchen 6 nach der letzten Strahlablenkeinheit 30d senkrecht zu der ursprünglichen Achse 14 des Strahls geladener Teilchen 6 verläuft und die ursprüngliche Achse 14 kreuzt.

Durch die beschriebene Ausbildung der Strahlablenkeinheiten 30a, 30b, 30c, 30d können verbesserte Strahleigenschaften bei einem gleichzeitig kompakteren Strahlführungssystem 2 erreicht werden. Dies wird unter anderem darauf zurückgeführt, dass die magnetischen Strahlablenkeinheiten 30a, 30b, 30c, 30d nicht nur ein Ablenken des Strahls geladener Teilchen sondern zudem auch eine Fokussierung des Strahls geladener Teilchen ähnliche einem Quadrupolmagneten erreichen können.

Die vorgesehenen magnetischen Strahlablenkeinheiten 30a, 30b, 30c, 30d weisen dabei eine Defokussierung in einer transversalen Richtung (hier in der Zeichenebene) und eine Fokussierung in einer dazu senkrechten Richtung auf. In diesem Punkt haben die magnetischen Strahlablenkeinheiten 30a, 30b, 30c, 30d ähnliche Eigenschaften wie Quadrupolmagnete, die ebenfalls in einer transversalen Richtung fokussieren und in der dazu senkrechten Richtung defokussieren. Alle vier magnetischen Strahlablenkeinheiten 30a, 30b, 30c, 30d fokussieren also nur in einer Richtung (senkrecht zur Zeichenebene). Von den vorgesehenen sieben Quadrupolmagneten 36a bis 36g (siehe unten) fokussieren daher fünf in der transversalen Richtung in der Zeichenebene und nur zwei in der dazu senkrechten Richtung. Insgesamt wird so eine ausreichende Fokussierung in beiden transversalen Koordinatenrichtungen erreicht. Im Endeffekt sind durch die Fokussierung der Dipole in y-Richtung also nur noch zwei Quadrupole mit Fokussierung in der gleichen Richtung erforderlich.

Zudem kann das Herstellungsverfahren der Strahlablenkeinheiten 30a, 30b, 30c, 30d aufgrund der parallelen Eintritts- und Austrittsseiten vereinfacht werden, da der Eisenkern der Strahlablenkeinheiten 30a, 30b, 30c, 30d durch parallel aufeinander geschichtete Platten hergestellt werden kann.

Das Strahlführungssystem 2 weist zudem mehrere, in diesem Fall sieben, zusätzliche magnetische Strahlformungseinheiten 36a, 36b, 36c, 36d, 36e, 36f, 36g in Form von Quadrupolmagneten auf. Durch die Strahlformungseinheiten 36a, 36b, 36c, 36d, 36e, 36f, 36g kann die Strahleigenschaft des Strahls geladener Teilchen 6 weiter verbessert werden. Insbesondere ist unter anderem aufgrund der Solenoid-Magnete 16a, 16b und der Strahlablenkeinheiten 30a, 30b, 30c, 30d nur eine vergleichsweise geringe Anzahl von zusätzlichen Strahlformungseinheiten 36a, 36b, 36c, 36d, 36e, 36f, 36g notwendig, um gute Strahleigenschaften zu erzielen, was ein kompaktes Strahlführungssystem 2 erlaubt.

Zwischen der (in Richtung 12 des Strahls geladener Teilchen 6 gesehen) ersten magnetischen Strahlablenkeinheit 30a und der zweiten magnetischen Strahlablenkeinheit 30b sind die fünf zusätzlichen Strahlformungseinheiten 36a, 36b, 36c, 36d, 36e vorgesehen. Zwischen der zweiten magnetischen Strahlablenkeinheit 30b und der dritten magnetischen Strahlablenkeinheit 30c sind weitere zwei zusätzliche Strahlformungseinheiten 36f, 36g vorgesehen. Die zusätzlichen Strahlformungseinheiten 36a, 36b, 36c, 36d, 36e, 36f, 36g sind in diesem Fall alle gleich dimensioniert.

Das Strahlführungssystem 2 weist zwischen den zwei magnetischen Strahlablenkeinheiten 30b, 30c eine weitere Kollimatoreinheit 38 in Form einer Blende mit einer rechteckige Öffnung 40 auf. Die Öffnung lässt sich in beide Richtungen transversal zum Strahl geladener Teilchen 6 verändern, wodurch eine Formgebung für den Strahlfleck am Behandlungsort 7 erfolgen kann. Es hat sich gezeigt, dass zwischen den Strahlablenkeinheiten 30b, 30c eine vergleichsweise große Impulsdispersion herrscht. Dieser kann durch das Vorsehen der Kollimatoreinheit 38 entgegengewirkt werden. Durch die Kollimatoreinheit 38 kann nämlich eine Impulsselektion für den Strahl geladener Teilchen 6 am Behandlungsort 7 erreichet werden.

In dem Strahlführungssystem 2 sind weiterhin die Strahlmonitore 42 und 44 vorgesehen. Der Strahlmonitor 42 ist zwischen den zusätzlichen Strahlformungseinheiten 36b und 36c vorgesehen. Der Strahlmonitor 44 ist nach der vierten Strahlablenkeinheit 30d und vor dem Behandlungszentrum 7 vorgesehen.

Weiterhin weist das Strahlführungssystem zwischen der Strahlablenkeinheit 30c und der Strahlablenkeinheit 30d einen Scanmagneten 46 auf. Der Scanmagnet kann vorteilhaft an dieser Position eingesetzt werden, da hierdurch am Behandlungsort ein großer Scanbereich abgedeckt werden kann. Mit einem System wie beispielsweise bereits 2005 von V. Anferov vorgestellt, lässt sich zum Beispiel der Strahl derart verschieben, dass am Behandlungsort ein Bereich von 210 mm mal 175 mm überdeckt werden kann, bei einem Ablenkwinkel von nur ± 44 mrad in beiden Koordinatenrichtungen. Eine weitere Vergrößerung des Scanbereichs ist möglich. Da die Strahlablenkeinheit 30d den durch den Scanmagneten 46 abgelenkten geladener Teilchen 6 aufnehmen muss, kann die Strahlablenkeinheit 30d eine im Vergleich zu den übrigen Strahlablenkeinheiten 30a, 30b, 30c vergrößerte Eintrittsöffnung und/oder Austrittsöffnung aufweisen. Die Strahlablenkeinheiten 30a, 30b, 30c können baugleich sein.

Dadurch, dass der Scanmagnet 46 zwischen der letzten und vorletzten (das heißt der dritten und der vierten) Strahlablenkeinheit 30c, 30d vorgesehen ist, kann ein großer Scan-Bereich am Behandlungsort 7 ermöglicht werden.

Es hat sich gezeigt, dass das Strahlführungssystem 2 besonders kompakt vorgesehen werden kann. Die Strecke 50 von der Strahlerzeugungseinheit 4 bis zum Ende der Energiekorrektureinheit 18 beträgt hier weniger als 2 m. Die Strecke 52 vom Ende der Energiekorrektureinheit 18 bis zum Behandlungsort 7 beträgt hier weniger als 10 m. Der Strahl geladener Teilchen 6 kann hier mit dem Strahlführungssystem 2 auf einer Strecke 54 von weniger als 8 m von der Richtung 12 entlang der ursprünglichen Achse 14 des Strahl geladener Teilchen 6 zum Behandlungsort 7 geführt werden. Dabei beträgt der maximale Abstand 56 des Strahls geladener Teilchen 6 weniger als 3 m von der ursprünglichen Achse 14 des Strahls geladener Teilchen 6. Der Abstand 58 der zweiten und dritten magnetischen Strahlablenkeinheit 30b, 30c beträgt dabei weniger als 1,5 m. Der Abstand 60 der letzten magnetische Strahlablenkeinheit 30d vom Behandlungsort beträgt dabei weniger als 1 m, beispielsweise 0,991m.

Zu beachten ist, dass sich die geometrischen Abmessungen auf einen Strahl geladener Teilchen mit einer kinetischen Energie von etwa 210MeV beziehen. Werden höhere Energien verwendet, werden die geometrischen Abmessungen bevorzugt mit einem Faktor multipliziert. Dieser geometrische Skalenfaktor ist beispielsweise gerade das Verhältnis der Impulse von Protonen höherer Energie (zum Beispiel 245 MeV) zu 210 MeV Protonen.

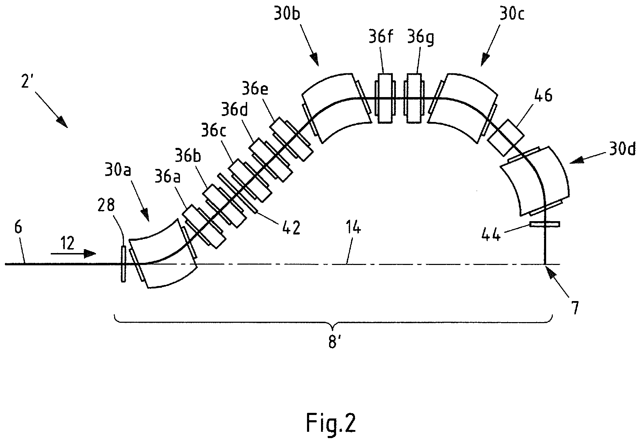

Fig. 2 zeigt eine schematische Schnittansicht eines weiteren Ausführungsbeispiels des beweglichen Abschnitts eines Strahlführungssystems 2'. Der gezeigte rotierbare Abschnitt 8' des Strahlführungssystems 2' aus Fig. 2 ist ähnlich zu dem rotierbaren Abschnitt 8 des Strahlführungssystems 2 aus Fig. 1. Insofern werden für gleiche Elemente gleiche Bezugszeichen verwendet. Ebenfalls wird auf die Ausführungen zu dem Strahlführungssystem 2 aus Fig. 1 verwiesen. Insbesondere kann der rotierbare Abschnitt 8' anstelle des rotierbaren Abschnitts 8 in dem Strahlführungssystem 2 vorgesehen werden. Im Folgenden soll lediglich auf die Unterschiede zu dem Strahlführungssystem 2 aus Fig. 1 eingegangen werden.

Der wesentliche Unterschied zwischen dem Strahlführungssystem 2 und dem Strahlführungssystem 2' ist, dass das Strahlführungssystem 2' keine Kollimatoreinheiten 24, 38 in dem rotierbaren Abschnitt 8' aufweist. Dadurch kann insbesondere der Abstand der magnetischen Strahlablenkeinheiten 30b, 30c verkürzt werden, sodass deren Abstand beispielsweise nur noch weniger als 1,2 m, insbesondere weniger als 1,1 m betragen kann. Die Selektion des Phasenraums erfolgt in diesem Fall bereits vor Eintritt des Strahls geladener Teilchen 6 in den rotierbaren Abschnitt 8'.

Fig. 3 zeigt eine schematische Schnittansicht eines Ausführungsbeispiels einer Energiekorrektureinheit 18 in Form eines Degraders, wie er beispielsweise in dem Strahlführungssystem 2 oder 2' verwendet werden kann. Die Energiekorrektureinheit 18 weist eine Mehrzahl von quer zum Strahl geladener Teilchen 6 verschiebbaren blockförmigen Energiekorrekturelementen 62a, 62b, 62c, 62d, 62e und zwei quer zum Strahl geladener Teilchen 6 verschiebbare keilförmige Energiekorrekturelemente 64a, 64b auf.

Die blockförmigen Energiekorrekturelemente 62a, 62b, 62c, 62d, 62e sind hier entlang des Pfeils 66 senkrecht zum Strahl geladener Teilchen 6 verschiebbar. Dadurch können unterschiedliche Einstellungen der Energie der geladenen Teilchen des Strahls geladener Teilchen 6 ermöglicht werden, je nachdem welches der blockförmigen Energiekorrekturelemente 62a, 62b, 62c, 62d, 62e in den Strahl geladener Teilchen geschoben wird. Die blockförmigen Energiekorrekturelemente 62a, 62b, 62c, 62d, 62e weisen hierzu in Richtung 12 des Strahls geladener Teilchen 6 gesehen unterschiedliche Ausdehnungen auf. Die blockförmigen Energiekorrekturelemente 62a, 62b, 62c, 62d, 62e dienen hier einer groben Einstellung der Energie der geladenen Teilchen des Strahls geladener Teilchen 6, indem die Energie der geladenen Teilchen des Strahls geladener Teilchen 6 durch die blockförmigen Energiekorrekturelemente 62a, 62b, 62c, 62d, 62e auf diskrete Werte eingestellt werden kann.

Die keilförmigen Energiekorrekturelemente 64a, 64b sind entlang der Pfeile 68 ebenfalls senkrecht zum Strahl geladener Teilchen 6 verschiebbar. Die keilförmigen Energiekorrekturelement 64a, 64b dienen einer Feineinstellung der Energie der geladenen Teilchen des Strahls geladener Teilchen 6 nachdem der Strahl geladener Teilchen 6 durch eines der blockförmigen Energiekorrekturelemente 62a, 62b, 62c, 62d, 62e geführt wurde. Die Energie der geladenen Teilchen des Strahls geladener Teilchen 6 kann durch die keilförmigen Energiekorrekturelement 64a, 64b in einem begrenzten Bereich kontinuierlich eingestellt werden. Durch die Verschiebung der keilförmigen Energiekorrekturelemente quer zum Strahl geladener Teilchen wird die in Richtung des Strahls geladener Teilchen 6 gesehene Ausdehnung des keilförmigen Energiekorrekturelements 64a, 64b im Bereich des Strahls geladener Teilchen verändert. Die zwei keilförmigen Energiekorrekturelemente 64a, 64b sind vorliegend punktsymmetrisch zueinander angeordnet. Die angeschrägten Flächen der keilförmigen Energiekorrekturelemente 64a, 64b sind einander zugewandt. Durch diese Anordnung kann eine über den Querschnitt des Strahls geladener Teilchen 6 asymmetrische Reduktion der Energie vermieden werden.

Die blockförmigen Energiekorrekturelemente 62a, 62b, 62c, 62d, 62e der Energiekorrektureinheit 18 sind aus Graphit und/oder aus Borcarbid hergestellt. Ebenfalls ist jedoch denkbar, blockförmige Energiekorrekturelemente unterschiedlicher Materialien vorzusehen. Auch die keilförmigen Energiekorrekturelemente 64a, 64b der Energiekorrektureinheit 18 sind aus Graphit und/oder aus Borcarbid hergestellt. Auch hier ist denkbar, keilförmige Energiekorrekturelemente unterschiedlicher Materialien vorzusehen.

Aufgrund der Kompaktheit der Energiekorrektureinheit 18 kann ein übermäßiges Aufweiten des Phasenraums des Strahls geladener Teilchen 6 vermieden werden.

In Richtung 12 des Strahls geladener Teilchen 6 gesehen hinter der Energiekorrektureinheit 18 ist ein Kollimator 20 vorgesehen. Dieser kann beispielsweise der in Fig. 1 gezeigte Kollimator sein.

Zusammenfassend ist es möglich, mittels kleiner Ablenkwinkel, verursacht durch das Magnetfeld des Scanmagneten 46, am Behandlungsort 7 einen großen Scanbereich zu erreichen und gleichzeitig die Abstände aller magnetischen Elemente von der Rotationsachse 14 gering zu halten.

Die Leistungsfähigkeit der Teilchenstrahl-Therapieanlage aus Fig. 1 kann mittels genauer numerischer Monte-Carlo-Berechnungen ermittelt werden. Beispielsweise beträgt die Transmissionseffizienz am Behandlungsort 7 bei Abbremsung einer von einem Zyklotron emittierten Energie von 215 MeV auf 70 MeV noch etwa 3,3 %. Das heißt man benötig nur einen Zyklotronstrom von einigen 10 nA, um einen Strom von 1 nA am Behandlungszentrum zu erreichen. Bei einer Abbremsung auf 90 MeV ist die Transmission bei der Verwendung von supraleitenden Solenoid-Magneten beispielsweise fünfmal höher als bei der Verwendung von Quadrupolmagneten. Die Breite des Strahlflecks kann dabei auf 3,6 mm (1σ) eingestellt werden bei sehr geringer Elliptizität (etwa 3 - 5%). Die Impulsbreite der am Behandlungsort 7 eintreffenden geladenen Teilchen lässt sich zwischen etwa 4 und 9 Promille variieren. Dabei bleibt die Größe des Strahlflecks am Behandlungsort 7 erhalten. Es hat sich gezeigt, dass bei einer Abbremsung durch die Energiekorrektureinheit 18 auf 90 MeV sogar eine Transmissionseffizienz bis zum Behandlungsort von bis zu 10% erreicht werden kann.

Für obige Ergebnisse wurde angenommen, dass die Magnetfelder der Solenoid-Magneten konstant für Teilchenenergien von 70 MeV bis 200 MeV sind. Der erste Solenoid 16a erzeugt ein Magnetfeld von 11.2 Tesla mit einer kalten Bohrung von 30 mm. Der zweite Solenoid 16b erzeugt ein Magnetfeld von 6.16 Tesla mit einer kalten Bohrung von 52 mm. Als Material für die Energiekorrektureinheit 18 wurde Borcarbid angenommen. Die Magnetfelder der Quadrupolmagnete skalieren für Energien ≤ 120 MeV mit dem mittleren Impuls des abgebremsten Strahls.

Die physikalischen Grundlagen der Wechselwirkung von Protonen mit Materie und die zur Berechnung der Eigenschaften von Strahlführungssystemen verwendeten Programme sind in den folgenden wissenschaftlichen Berichten beschrieben:
- Particle Data Group, W.-M. Yao et al., "The Review of Particle Physics", Journal of Physics G33 (2006) 1 and update 2008.
- Karl L. Brown, Sam K. Howry, "TRANSPORT, A Computer Program for Designing Charged Particle Beam Transport Systems", SLAC Report No. 91 (1970), SLAC Report 91, Rev. 3 (1983) and later updates of the TRANSPORT program by U. Rohrer and others.
- U. Rohrer, "PSI Graphic TURTLE Framework based on a CERN-SLAC-FERMILAB version by K.L. Brown et al.", http://aea.web.psi.ch/Urs_Rohrer/MyWeb/turtle.htm.
- J. Drees, "Passage of Protons through Thick Degraders", Cryoelectra Report Sept. 2008.

Das Prinzip eines x-y Scan-Magneten ist in folgendem wissenschaftlichen Bericht beschrieben:
- V. Anferov, "Combined X-Y scanning magnet for conformal proton radiation therapy", Med. Phys. 32 (3), March 2005.

## Patentansprüche

1. Teilchenstrahl-Therapieanlage (1)
- mit einer Strahlerzeugungseinheit (4) zur Erzeugung eines Strahls geladener Teilchen (6), insbesondere Ionen, vorzugsweise Protonen, und
- mit einem Strahlführungssystem (2, 2'), wobei das Strahlführungssystem (2, 2') einen unbeweglichen Abschnitt (10) und einen beweglichen, insbesondere rotierbaren Abschnitt (8) aufweist, wobei das Stahlführungssystem in Richtung (12) des Strahls geladener Teilchen (6) gesehen hinter der Strahlerzeugungseinheit (4) mindestens einen Solenoid-Magneten (16a, 16b) als Strahlformungseinheit aufweist, und der mindestens eine Solenoid-Magnet (16a, 16b) des Stahlführungssystems (2, 2') ein supraleitender Solenoid-Magnet (16a, 16b) ist,
**dadurch gekennzeichnet, dass**
der mindestens eine Solenoid-Magnet (16a, 16b) im unbeweglichen Abschnitt (10) des Strahlführungssystems (2, 2') vorgesehen ist.

2. Teilchenstrahl-Therapieanlage (1) nach Anspruch 1,
wobei mindestens ein Solenoid-Magnet (16a, 16b) des Strahlführungssystems (2, 2') unmittelbar hinter der Strahlerzeugungseinheit (4) vorgesehen ist.

3. Teilchenstrahl-Therapieanlage (1) nach Anspruch 1 oder 2,
wobei das Strahlführungssystem (2, 2') eine Energiekorrektureinheit (18) zur Einstellung der Energie der geladenen Teilchen des Strahls geladener Teilchen (6) aufweist und mindestens ein Solenoid-Magnet (16a, 16b) des Strahlführungssystems (2, 2') in Richtung (12) des Strahls geladener Teilchen (6) gesehen zwischen der Strahlerzeugungseinheit (4) und der Energiekorrektureinheit (18) vorgesehen ist und/oder mindestens ein Solenoid-Magnet (16a, 16b) des Strahlführungssystems (2, 2') in Richtung (12) des Strahls geladener Teilchen (6) gesehen hinter der Energiekorrektureinheit (18) vorgesehen ist.

4. Teilchenstrahl-Therapieanlage (1) nach einem der Ansprüche 1 bis 3,
wobei das Strahlführungssystem (2, 2') mindestens zwei Solenoid-Magnete (16a, 16b) in Richtung (12) des Strahls geladener Teilchen (6) gesehen hinter der Strahlerzeugungseinheit (4) aufweist.

5. Teilchenstrahl-Therapieanlage (1) nach einem der Ansprüche 1 bis 4,
wobei mindestens ein Solenoid-Magnet (16a, 16b) als eine im Wesentlichen geradlinig verlaufende Zylinderspule ausgebildet ist.

6. Teilchenstrahl-Therapieanlage (1) nach einem der Ansprüche 1 bis 5,
wobei die Wicklungen mindestens eines Solenoid-Magneten (16a, 16b) im Wesentlichen senkrecht zum Strahl geladener Teilchen (6) verlaufen.

7. Teilchenstrahl-Therapieanlage (1) nach einem der Ansprüche 1 bis 6,
wobei mindestens ein Solenoid-Magnet (16a, 16b) zumindest abschnittsweise ein im Wesentlichen homogenes Magnetfeld erzeugt.

8. Teilchenstrahl-Therapieanlage (1) nach einem der Ansprüche 1 bis 7,
wobei das Magnetfeld mindestens eines Solenoid-Magneten (16a, 16b) im Bereich von 1 Tesla bis 10 Tesla, vorzugsweise im Bereich von 4 Tesla bis 8 Tesla liegt und/oder das Magnetfeld mindestens eines Solenoid-Magneten (16a, 16b) im Bereich von 5 Tesla bis 20 Tesla, vorzugsweise im Bereich von 8 Tesla bis 15 Tesla liegt.

9. Teilchenstrahl-Therapieanlage (1) nach einem der Ansprüche 1 bis 8,
wobei die Teilchenstrahl-Therapieanlage (1) derart eingerichtet ist, dass mindestens ein Solenoid-Magnet (16a, 16b) bei unterschiedlichen Energien der geladenen Teilchen des Strahls geladener Teilchen (6) ein im Wesentlichen konstantes Magnetfeld erzeugt.

10. Teilchenstrahl-Therapieanlage (1) nach einem der Ansprüche 1 bis 9,
wobei mindestens ein Solenoid-Magnet (16a, 16b) eine Eintrittsöffnung und/oder Austrittsöffnung zwischen 10 mm und 50 mm, vorzugsweise zwischen 20 mm und 40 mm aufweist und/oder mindestens ein Solenoid-Magnet (16a, 16b) eine Eintrittsöffnung und/oder Austrittsöffnung zwischen 30 mm und 70 mm, vorzugsweise zwischen 40 mm und 60 mm aufweist.

11. Teilchenstrahl-Therapieanlage (1) nach einem der Ansprüche 1 bis 10,
wobei das Strahlführungssystem (2, 2') mindestens eine magnetische Strahlablenkeinheit (30a, 30b, 30c, 30d), insbesondere im beweglichen Abschnitt (8) des Strahlführungssystems (2, 2'), aufweist.

12. Teilchenstrahl-Therapieanlage (1) nach Anspruch 11,
wobei mindestens ein Solenoid-Magnet (16a, 16b) in Richtung (12) des Strahls geladener Teilchen (6) gesehen vor der mindestens einen magnetischen Strahlablenkeinheit (30a, 30b, 30c, 30d) angeordnet ist.

13. Teilchenstrahl-Therapieanlage (1) nach einem der Ansprüche 1 bis 12,
wobei das Strahlführungssystem (2, 2') mindestens eine zusätzliche magnetische Strahlformungseinheit (36a, 36b, 36c, 36d, 36e, 36f, 36g), insbesondere in Form eines Quadrupolmagneten, insbesondere im beweglichen Abschnitt (8) des Strahlführungssystems (2, 2'), aufweist.

14. Verfahren, ausgeführt mit einer Teilchenstrahl-Therapieanlage nach einem der Ansprüche 1 bis 13, umfassend die Schritte:
- Erzeugen eines Strahls geladener Teilchen, insbesondere Ionen, vorzugsweise Protonen, mit der Strahlerzeugungseinheit, und
- Führen des Strahls geladener Teilchen mittels des Strahlführungssystems.

## Claims

1. A particle beam treatment system (1)
- with a beam generation unit (4) for generating a beam of charged particles (6), in particular ions, preferably protons, and
- with a beam guidance system (2, 2'),wherein the beam guidance system (2, 2') has an immovable section (10) and a movable, in particular rotatable, section (8), wherein the beam guidance system seen in the direction (12) of the beam of charged particles (6) behind the beam generation unit (4) has at least one solenoid magnet (16a, 16b) as a beam shaping unit, and the at least one solenoid magnet (16a, 16b) of the beam guidance system (2, 2') is a superconducting solenoid magnet (16a, 16b),
**characterized in that** the at least one solenoid magnet (16a, 16b) is provided in the immovable section (10) of the beam guidance system (2, 2').

2. A particle beam treatment system (1) according to claim 1,
wherein at least one solenoid magnet (16a, 16b) of the beam guidance system (2, 2') is provided directly behind the beam generation unit (4).

3. A particle beam treatment system (1) according to claim 1 or 2,
wherein the beam guidance system (2, 2') has an energy correction unit (18) for adjusting the energy of the charged particles of the beam of charged particles (6) and at least one solenoid magnet (16a, 16b) of the beam guidance system (2, 2') seen in the direction of (12) of the beam of charged particles (6) is provided between the beam generation unit (4) and the energy correction unit (18) and/or at least one solenoid magnet (16a, 16b) of the beam guidance system (2, 2') seen in the direction of (12) of the beam of charged particles (6) is provided behind the energy correction unit (18).

4. A particle beam treatment system (1) according to one of claims 1 to 3,
wherein the beam guidance system (2, 2') has at least two solenoid magnets (16a, 16b) seen in the direction of (12) of the beam of charged particles (6) behind the beam generation unit (4).

5. A particle beam treatment system (1) according to one of claims 1 to 4,
wherein at least one solenoid magnet (16a, 16b) is designed as a cylindrical coil running in a substantially linear direction.

6. A particle beam treatment system (1) according to one of claims 1 to 5,
wherein the windings of at least one solenoid magnet (16a, 16b) run substantially perpendicularly to the beam of charged particles (6).

7. A particle beam treatment system (1) according to one of claims 1 to 6,
wherein at least one solenoid magnet (16a, 16b) generates at least in sections a substantially homogenous magnetic field.

8. A particle beam treatment system (1) according to one of claims 1 to 7,
wherein the magnetic field of at least one solenoid magnet (16a, 16b) is in the range of 1 tesla to 10 tesla, preferably in the range of 4 tesla to 8 tesla and/or the magnetic field of at least one solenoid magnet (16a, 16b) is in the range of 5 tesla to 20 tesla, preferably in the range of 8 tesla to 15 tesla.

9. A particle beam treatment system (1) according to one of claims 1 to 8,
wherein the particle beam treatment system (1) is configured so that at least one solenoid magnet (16a, 16b) in the case of differing energies of the charged particles (6) of the beam of charged particles generates a substantially constant magnetic field.

10. A particle beam treatment system (1) according to one of claims 1 to 9,
wherein at least one solenoid magnet (16a, 16b) has an entry opening and/or exit opening of between 10 mm and 50 mm, preferably of between 20 mm and 40 mm and/or at least one solenoid magnet (16a, 16b) has an entry opening and/or exit opening of between 30 mm and 70 mm, preferably of between 40 mm and 60 mm.

11. A particle beam treatment system (1) according to one of claims 1 to 10,
wherein the beam guidance system (2, 2') has at least one magnetic beam deflection unit (30a, 30b, 30c, 30d), in particular in the movable section (8) of the beam guidance system (2, 2').

12. A particle beam treatment system (1) according to claim 11,
wherein at least one solenoid magnet (16a, 16b) seen in the direction of (12) of the beam of charged particles (6) is disposed before the at least one magnetic beam deflection unit (30a, 30b, 30c, 30d).

13. A particle beam treatment system (1) according to one of claims 1 to 12,
wherein the beam guidance system (2, 2') has at least one additional magnetic beam shaping unit (36a, 36b, 36c, 36d, 36e, 36f, 36g), in particular in the form of a quadrupole magnet, in particular in the movable section (8) of the beam guidance system (2, 2').

14. A method, carried out with a particle beam treatment system according to one of claims 1 to 13, comprising the steps of:
- generating a beam of charged particles, in particular ions, preferably protons, with the beam generation unit, and
- guiding the beam of charged particles by means of the beam guidance system.

## Revendications

1. Installation thérapeutique à faisceau de particules (1)
- avec une unité de production de faisceau (4) pour produire un faisceau de particules chargées (6), notamment d'ions, de préférence de protons, et
- avec un système de guidage de faisceau (2, 2'), le système de guidage de faisceau (2, 2') comportant un tronçon immobile (10) et un tronçon mobile, notamment rotatif (8),
le système de guidage de faisceau vu en direction (12) du faisceau de particules chargées (6) comportant derrière l'unité de production de faisceau (4) au moins un aimant à solénoïde (16a, 16b) en tant qu'unité de formation de faisceau, et
au moins un aimant à solénoïde (16a, 16b) du système de guidage de faisceau (2,2') étant un aimant à solénoïde supraconducteur (16a, 16b),
**caractérisée en ce qu'**au moins un aimant à solénoïde (16a, 16b) est prévu dans le tronçon immobile (10) du système de guidage de faisceau (2, 2').

2. Installation thérapeutique à faisceau de particules (1) selon la revendication 1, au moins un aimant à solénoïde (16a, 16b) du système de guidage de faisceau (2, 2') étant prévu directement derrière l'unité de production de faisceau (4).

3. Installation thérapeutique à faisceau de particules (1) selon la revendication 1 ou 2, le système de guidage de faisceau (2, 2') comportant une unité de correction d'énergie (18) pour régler l'énergie des particules chargées du faisceau de particules chargées (6) et au moins un aimant à solénoïde (16a, 16b) du système de guidage de faisceau (2, 2'), vu en direction (12) du faisceau de particules chargées (6), étant prévu entre l'unité de production de faisceau (4) et l'unité de correction d'énergie (18) et/ou au moins un aimant à solénoïde (16a, 16b) du système de guidage de faisceau (2, 2'), vu en direction (12) du faisceau de particules chargées (6) étant prévu derrière l'unité de correction d'énergie (18).

4. Installation thérapeutique à faisceau de particules (1) selon l'une quelconque des revendications 1 à 3, le système de guidage de faisceau (2, 2') comportant au moins deux aimants à solénoïde (16a, 16b) vus en direction (12) du faisceau de particules chargées (6), derrière l'unité de production de faisceau (4).

5. Installation thérapeutique à faisceau de particules (1) selon l'une quelconque des revendications 1 à 4, au moins un aimant à solénoïde (16a, 16b) étant constitué comme une bobine cylindrique passant pour l'essentiel en ligne droite.

6. Installation thérapeutique à faisceau de particules (1) selon l'une quelconque des revendications 1 à 5, les enroulements d'au moins un aimant à solénoïde (16a, 16b) passant pour l'essentiel perpendiculairement au faisceau de particules chargées (6).

7. Installation thérapeutique à faisceau de particules (1) selon l'une quelconque des revendications 1 à 6, au moins un aimant à solénoïde (16a, 16b) produisant au moins par section un champ magnétique pour l'essentiel homogène.

8. Installation thérapeutique à faisceau de particules (1) selon l'une quelconque des revendications 1 à 7, le champ magnétique d'au moins un aimant à solénoïde (16a, 16b) se situant dans la zone de 1 Tesla à 10 Tesla, de préférence dans la zone de 4 Tesla à 8 Tesla et/ou le champ magnétique d'au moins un aimant à solénoïde (16a, 16b) se situant dans la zone de 5 Tesla à 20 Tesla, de préférence dans la zone de 8 Tesla à 15 Tesla.

9. Installation thérapeutique à faisceau de particules (1) selon l'une quelconque des revendications 1 à 8, l'installation thérapeutique à faisceau de particules (1) étant agencée de telle manière qu'au moins un aimant à solénoïde (16a, 16b) produit à des énergies différentes des particules chargées du faisceau de particules chargées (6), un champ magnétique pour l'essentiel constant.

10. Installation thérapeutique à faisceau de particules (1) selon l'une quelconque des revendications 1 à 9, au moins un aimant à solénoïde (16a, 16b) comportant une ouverture d'entrée et/ou une ouverture de sortie entre 10 mm et 50 mm, de préférence entre 20 mm et 40 mm et/ou au moins un aimant à solénoïde (16a, 16b) comportant une ouverture d'entrée et/ou une ouverture de sortie entre 30 mm et 70 mm, de préférence entre 40 mm et 60 mm.

11. Installation thérapeutique à faisceau de particules (1) selon l'une quelconque des revendications 1 à 10, le système de guidage de faisceau (2, 2') comportant au moins une unité de déflexion de faisceau (30a, 30b, 30c, 30d), notamment dans le tronçon mobile (8) du système de guidage du faisceau (2, 2').

12. Installation thérapeutique à faisceau de particules (1) selon la revendication 11, au moins un aimant à solénoïde (16a, 16b) vu en direction (12) du faisceau de particules chargées (6) étant disposé devant au moins une unité de déflexion de faisceau magnétique (30a, 30b, 30c, 30d).

13. Installation thérapeutique à faisceau de particules (1) selon l'une quelconque des revendications 1 à 12, le système de guidage de faisceau (2, 2') comportant au moins une unité de formation de faisceau magnétique supplémentaire (36a, 36b, 36c, 36d, 36e, 36f, 36g), notamment sous la forme d'un aimant quadripolaire, notamment dans le tronçon mobile (8) du système de guidage de faisceau (2, 2').

14. Procédé, exécuté avec une installation thérapeutique à faisceau de particules selon l'une quelconque des revendications 1 à 13, comprenant les étapes :
- de production d'un faisceau de particules chargées, notamment d'ions, de préférence de protons, avec une unité de production de faisceau, et
- De guidage du faisceau de particules chargées au moyen d'un système de guidage de faisceau.
